(19) Europäisches Patentamt
European Patent Office
Office européen des brevets

(11) Publication number: **0 624 563 A1**

(12) **EUROPEAN PATENT APPLICATION**

(21) Application number: **94107283.7**

(22) Date of filing: **10.05.94**

(51) Int. Cl.[5]: **C07C 41/01**, C07C 41/28, C07C 43/13, C07D 317/22

(30) Priority: **13.05.93 JP 111813/93**
**13.05.93 JP 111814/93**
**13.05.93 JP 111815/93**

(43) Date of publication of application:
**17.11.94 Bulletin 94/46**

(84) Designated Contracting States:
**DE FR GB**

(71) Applicant: **KAO CORPORATION**
**14-10, Nihonbashi Kayaba-cho 1-chome**
**Chuo-ku Tokyo (JP)**

(72) Inventor: **Nakagawa, Shoji**
**301, 3-30, Nishikoniri 3-chome**
**Wakayama-shi, Wakayama (JP)**
Inventor: **Yokota, Yuikinaga**
**208-10, Tottorinaka**
**Hannan-shi, Osaka (JP)**

(74) Representative: **Hansen, Bernd, Dr.**
**Dipl.-Chem. et al**
**Hoffmann, Eitle & Partner,**
**Patentanwälte,**
**Arabellastrasse 4**
**D-81925 München (DE)**

(54) **Glycerin derivatives and process for producing the same.**

(57) Glycerine derivatives represented by formula (1A), (2A) and (3A):

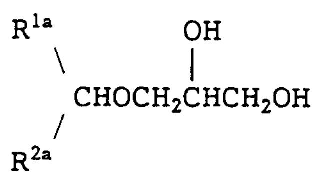

(1A)

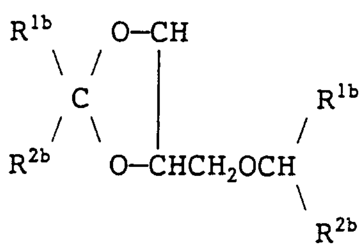

(2A)

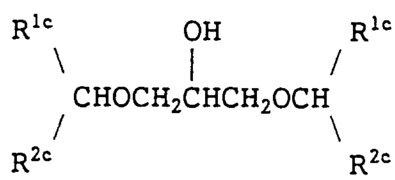

(3A)

wherein $R^{1a}$, $R^{2a}$, $R^{1b}$, $R^{2b}$, $R^{1c}$, and $R^{2c}$ are as defined in the disclosure, and process for producing glycerin

EP 0 624 563 A1

derivatives, including the glycerin derivatives (1A), (2A) and (3A), are disclosed. The glycerin derivatives have satisfactory physical properties and are applicable as lubricants or polar oils and also have mutual effects with water and are applicable as emulsifying agents or moisture retaining agents. The process makes it possible to synthesize glycerin derivatives from easily and economically available aldehydes or ketones in high yields.

## FIELD OF THE INVENTION

This invention relates to glycerin derivatives useful as oily stain dissolving agents, water-soluble organic solvents, polar oils, emulsifying agents, lubricating agents, moisturizing agents, and intermediates for surfactants and a process for producing the same.

## BACKGROUND OF THE INVENTION

Alkyl glyceryl ethers have been employed in wide fields, such as fixing agents for perfumes (see U.S. Patent 2,091,162), soap additives (see U.S. Patents 2,157,022 and 3,350,460), extracting solvents for organic substances (see U.S. Patent 2,156,724), moisture retaining agents (see JP-A-3-14505 and JP-A-3-14506, the term "JP-A" as used herein means an "unexamined published Japanese patent application")), components of aqueous ink compositions (see JP-A-58-67770), and components of cosmetics (JP-A-52-12109, JP-B-57-36260, and JP-B-3-31187 (the term "JP-B" as used herein means an "examined published Japanese patent application").

As alkyl glyceryl ethers, palmityl glyceryl ether (chimyl alcohol) and stearyl glyceryl ether (batyl alcohol) which occur in lipid of fishes are conventionally known. These naturally occurring alkyl glyceryl ethers are in use in broad fields for their excellent performance as an emulsifying agent. However, since they are solids having a high melting point, their applications in compositions comprising multiple components, such as cosmetics, are restrained. Hence, synthetic glyceryl ethers with various alkyl groups have been developed for use in cosmetics and the like preparations. For example, lauryl glyceryl ether, $\beta$-branched alkyl glyceryl ethers (see JP-A-52-12109), a mixture of isostearyl glyceryl ethers having a methyl branch in nearly the middle of the alkyl chain thereof (see JP-B-57-36260), and glyceryl ethers of terpene alcohols (see JP-B-3-31187) have been proposed.

Known processes for producing these alkyl glyceryl ethers include a process comprising hydrolysis by way of 1,3-dioxolane, using an alkyl glycidyl ether synthesized from an alcohol and epichlorohydrin (see U.S. Patent 3,024,273 and JP-B-57-36260) as disclosed in JP-A-56-133281; and a process comprising direct hydrolysis as disclosed in JP-A-49-86307.

However, the conventional synthetic alkyl glyceryl ethers are mixed alkyl ethers, the quality of which is difficult to control. Additionally, because the raw materials of synthesis are of natural origin, limited in availability, so that the products become expensive.

Further, the conventional synthetic processes involve such problems that a large quantity of waste, such as by-produced sodium chloride, accompanies; and that the productivity is poor and the processing steps are complicated, resulting in increased cost of production.

## SUMMARY OF THE INVENTION

Accordingly, an object of the present invention is to provide a novel glycerine derivative having broadened applicability.

Another object of the present invention is to provide a process for producing such a glycerine derivative with industrial advantages.

The present inventors have conducted extensive investigations for obtaining glycerine derivatives from easily available raw materials. As a result, it has now been found that glycerine derivatives can industrially be obtained by starting with an aldehyde or a ketone and by way of an acetal or a ketal as an intermediate. It has also been found that the thus obtained glycerin derivatives include novel compounds which have a low melting point and are therefore useful as polar oils or components of cosmetics. The present invention has been completed based on these findings.

The present invention provides a process for producing a glycerine derivative represented by formula (1):

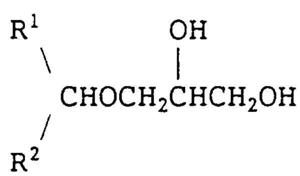

(1)

wherein $R^1$ represents a hydrogen atom or an alkyl group having from 1 to 21 carbon atoms; $R^2$ represents an alkyl group having from 1 to 21 carbon atoms; or $R^1$ and $R^2$ are taken together to form an alkylene group having from 2 to 21 carbon atoms; provided that $R^1$ and $R^2$ do not simultaneously represent an alkyl group having no hydrogen atom at the $\alpha$-position thereof,
comprising reacting a carbonyl compound represented by formula (4):

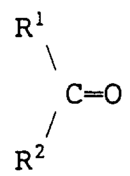

(4)

wherein $R^1$ and $R^2$ are as defined above,
with glycerin in the presence of an acid catalyst, and hydrogenating the resulting compound.

The present invention also relates to a glycerin derivative represented by formula (1A):

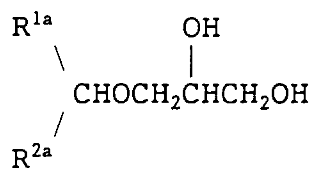

(1A)

wherein $R^{1a}$ and $R^{2a}$, which may be the same or different, each represent an alkyl group having from 2 to 21 carbon atoms.

The present invention further relates to a process for producing a glycerin derivative represented by formula (2):

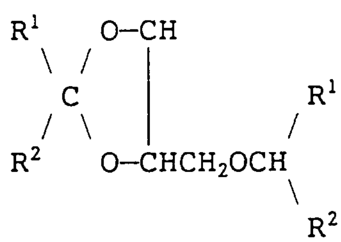

(2)

wherein $R^1$ and $R^2$ are as defined above,
comprising reacting a carbonyl compound represented by formula (4) with glycerin in the presence of an acid catalyst and hydrogenating the resulting compound.

The present invention furthermore relates to a glycerin derivative represented by formula (2A):

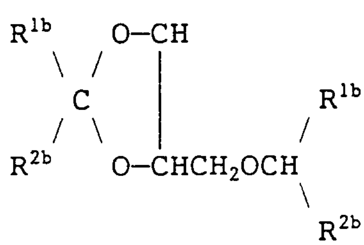

(2A)

wherein $R^{1b}$ and $R^{2b}$, which may be the same or different, each represent an alkyl group having from 3 to 21 carbon atoms.

The present invention yet relates to a process for producing a glycerin derivative represented by formula (3):

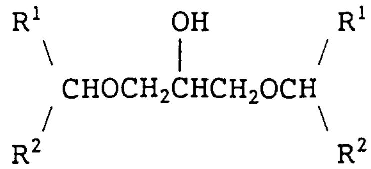

(3)

wherein $R^1$ and $R^2$ are as defined above,
comprising reacting a carbonyl compound represented by formula (4) with glycerin in the presence of an acid catalyst and hydrogenating the resulting compound.

The present invention moreover relates to a glycerin derivative represented by formula (3A):

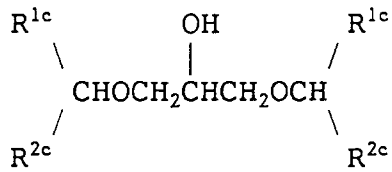

(3A)

wherein $R^{1c}$ represents an alkyl group having from 1 to 21 carbon atoms; and $R^{2c}$ represents an alkyl group having from 3 to 21 carbon atoms.

## DETAILED DESCRIPTION OF THE INVENTION

In formulae (1) to (4), the alkyl group having from 1 to 21 carbon atoms, represented by $R^1$ or $R^2$, includes straight-chain or branched alkyl groups, such as methyl, ethyl, n-propyl, isopropyl, n-butyl, isobutyl, sec-butyl, pentyl, hexyl, heptyl, 1-ethylpentyl, octyl, nonyl, decyl, undecyl, dodecyl, tridecyl, tetradecyl, pentadecyl, hexadecyl, heptadecyl, octadecyl, nonadecyl, eicosyl, heneicosyl, isoheptadecyl, 1-methyloctyl, 1-methyldecyl, and 2,4,4-trimethylpentyl groups. The alkylene group having from 2 to 21 carbon atoms, formed by $R^1$ and $R^2$, includes ethylene, trimethylene, tetramethylene, pentamethylene, 1-methylpentamethylene, 2-methylpentamethylene, 3-methylpentamethylene, 2,2,4-trimethylpentamethylene, 1-t-butylpentamethylene, 3-t-butylpentamethylene, hexamethylene, and heptamethylene groups.

In formula (1A), the alkyl group having from 2 to 21 carbon atoms, as represented by $R^{1a}$ or $R^{2a}$, includes straight-chain or branched alkyl groups, such as ethyl, n-propyl, isopropyl, n-butyl, isobutyl, sec-butyl, pentyl, hexyl, heptyl, 1-ethylpentyl, octyl, nonyl, decyl, undecyl, dodecyl, tridecyl, tetradecyl, pentadecyl, hexadecyl, heptadecyl, octadecyl, nonadecyl, eicosyl, heneicosyl, isoheptadecyl, 1-methyloctyl, and 1-methyldecyl groups.

In formulae (2A) and (3A), the alkyl group having from 3 to 21 carbon atoms, as represented by $R^{1b}$, $R^{2b}$ or $R^{2c}$, includes straight chain or branched alkyl groups, such as n-propyl, isopropyl, n-butyl, isobutyl, sec-butyl, pentyl, hexyl, heptyl, 1-ethylpentyl, octyl, nonyl, decyl, undecyl, dodecyl, tridecyl, tetradecyl, pentadecyl, hexadecyl, heptadecyl, octadecyl, nonadecyl, eicosyl, heneicosyl, isoheptadecyl, 1-methyloctyl, and 1-methyldecyl groups.

In formula (3A), the alkyl group having from 1 to 21 carbon atoms, as represented by $R^{1c}$, includes straight chain or branched alkyl groups, such as methyl, ethyl, n-propyl, isopropyl, n-butyl, isobutyl, sec-butyl, pentyl, hexyl, heptyl, 1-ethylpentyl, octyl, nonyl, decyl, undecyl, dodecyl, tridecyl, tetradecyl, pentadecyl, hexadecyl, heptadecyl, octadecyl, nonadecyl, eicosyl, heneicosyl, isoheptadecyl, 1-methyloctyl, and 1-methyldecyl groups.

The process according to the present invention is illustrated by the following reaction scheme:

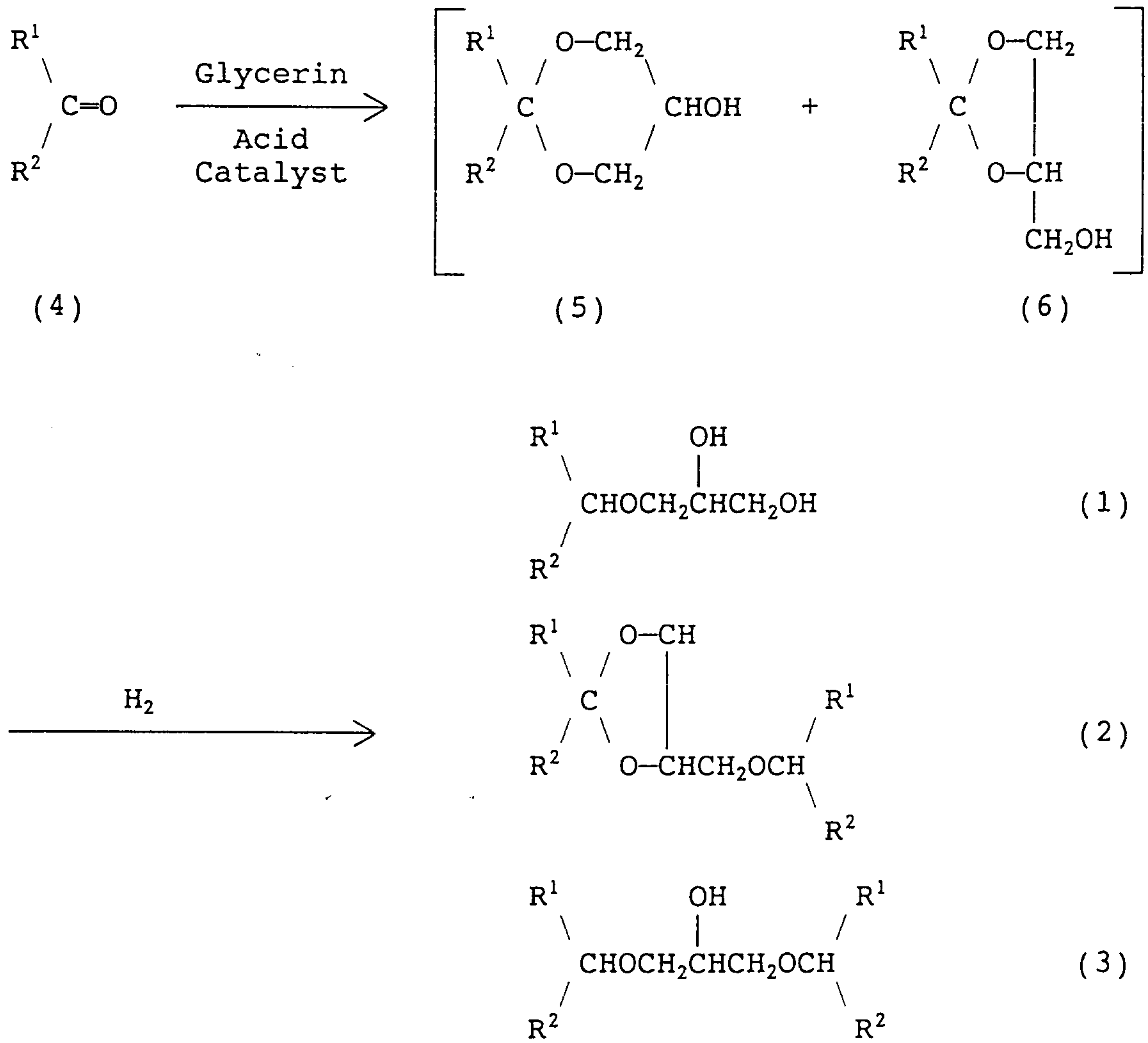


(4) (5) (6)

(1)

(2)

(3)

wherein $R^1$ and $R^2$ are as defined above.

The carbonyl compound (4) which can be used as a starting material includes an alkylaldehyde and a dialkyl ketone.

The alkylaldehyde is easily obtained by, for example, dehydrogenation of an aliphatic alcohol, hydroformylation of an olefin (oxo synthesis), Rosenmund reduction of a fatty acid chloride, or direct hydrogenation of a fatty acid. The product as obtained by the oxo synthesis is a mixture of a straight-chain alkylaldehyde and a branched alkylaldehyde, which can be separated into each component by precise distillation.

Specific but non-limiting examples of the alkylaldehydes include acetaldehyde, propionaldehyde, butyraldehyde, isobutyraldehyde, valeraldehyde, isovaleraldehyde, caproaldehyde, heptaldehyde, caprylic aldehyde, 2-ethylhexyl aldehyde, pelargonaldehyde, capric aldehyde, undecylic aldehyde, lauraldehyde, tridecylic aldehyde, myristyl aldehyde, pentadecylic aldehyde, palmityl aldehyde, margaric aldehyde, stearaldehyde, isostearic aldehyde, 2-methylnonyl aldehyde, 2-methylundecylic aldehyde, behenic aldehyde, and 3,5,5-trimethylhexyl aldehyde.

The dialkyl ketone can easily be obtained by a high-temperature decarbonating dimerization of a fatty acid, which is an economical oleochemical, catalytic oxidation of an olefin (Wacker process), oxidation or dehydrogenation of a secondary alcohol, or oxidation of a cycloalkane. The product as obtained by a Wacker process has a distribution but can be separated into each component by precise distillation.

Specific but non-limiting examples of the dialkyl ketones are acetone, methyl ethyl ketone, methyl propyl ketone, methyl isopropyl ketone, methyl butyl ketone, methyl isobutyl ketone, methyl pentyl ketone, methyl hexyl ketone, methyl heptyl ketone, methyl octyl ketone, methyl nonyl ketone, methyl decyl ketone, methyl undecyl ketone, methyl dodecyl ketone, methyl tridecyl ketone, methyl tetradecyl ketone, methyl pentadecyl ketone, methyl hexadecyl ketone, methyl heptadecyl ketone, methyl octadecyl ketone, methyl

nonadecyl ketone, methyl eicosyl ketone, methyl heneicosyl ketone, cyclohexanone, 2-methylcyclohexanone, 3-methylcyclohexanone, 4-methylcyclohexanone, 3,3,5-trimethylcyclohexanone, 2-t-butylcyclohexanone, 4-t-butylcyclohexanone, cyclopentanone, cyclobutanone, cyclopropanone, cyclododecanone, cycloheptanone, cyclooctanone, diethyl ketone, dipropyl ketone, dibutyl ketone, dipentyl ketone, dihexyl ketone, diheptyl ketone, dioctyl ketone, dinonyl ketone, diundecyl ketone, ditridecyl ketone, dipentadecyl ketone, diheptadecyl ketone, 3-hexanone, 3-heptanone, 3-octanone, 4-octanone, 3-nonanone, 4-nonanone, 3-decanone, 4-decanone, 3-undecanone, 4-undecanone, 5-undecanone, 3-dodecanone, 5-dodecanone, 3-tridecanone, diisopropyl ketone, and diisobutyl ketone.

In a first step of the process, the reaction of alkylaldehyde (4) with glycerin is an acetal-formation reaction. A glycerin to alkylaldehyde molar ratio is from 0.2 to 1.5, preferably from 0.6 to 1.2. The reaction is carried out in the presence of from 0.01 to 5 mol %, preferably from 0.1 to 1 mol %, based on alkylaldehyde (4), of an acid catalyst, such as p-toluenesulfonic acid, methanesulfonic acid or sulfuric acid with or without an inert solvent, such as xylene, toluene, benzene, octane, isooctane, heptane, hexane, cyclohexane, pentane, butane, ligroin or petroleum ether, or a mixed solvent thereof. While varying depending on the boiling point of the aldehyde used, the reaction is effected at a temperature of from 20 to 130 °C, preferably 50 to 100 °C, preferably while removing produced water. At too low temperatures, the reaction hardly proceeds. At too high temperatures, the reaction system tends to be considerably colored, and side reactions tend to take place. The reaction may be carried out in a nitrogen stream, in a nitrogen atmosphere, or in air. While depending on various conditions, the reaction time is usually from 1 to 30 hours. The reaction mixture is neutralized and subjected to pre-purification such as filtration, washing and the like. The recovered crude acetal is purified by a treatment with clay, crystal precipitation, distillation and the like operation.

The reaction between dialkylketone (4) and glycerin is a ketal-formation reaction. A glycerin to dialkyl ketone molar ratio is from 0.2 to 1.5, preferably from 0.6 to 1.2. The reaction is carried out in the presence of from 0.1 to 5 mol %, preferably from 1 to 3 mol %, based on dialkyl ketone (4), of an acid catalyst, such as p-toluenesulfonic acid, methanesulfonic acid or sulfuric acid, with or without an inert solvent, such as xylene, toluene, benzene, octane, isooctane, heptane, hexane, cyclohexane, pentane, butane, ligroin or petroleum ether, or a mixed solvent thereof. While varying depending on the boiling point of the ketone used, the reaction is effected at a temperature of from 40 to 130 °C, preferably 70 to 100 °C, preferably while removing produced water. At too low temperatures, the reaction hardly proceeds. At too high temperatures, the reaction system is considerably colored and side reactions tend to take place. The reaction may be in a nitrogen stream, in a nitrogen atmosphere, or in air. While depending on various conditions, the reaction time is usually from 5 to 200 hours. The reaction mixture is neutralized and subjected to pre-purification such as filtration, washing and the like. The recovered crude ketal is purified by a treatment with clay, precipitation, distillation, or the like operation.

In a second step of the process, acetal or ketal ((5) + (6)) is hydrogenated to provide a mixture comprising glyceryl derivatives of formulae (1), (2), and (3). The hydrogenation reaction is carried out in the presence of 5 to 5000 ppm, based on the acetal or ketal, of a conventional catalyst for hydrogenolysis, such as palladium, rhodium, ruthenium or platinum, under a hydrogen pressure of from atmospheric pressure to 250 $kg/cm^2$ at a temperature of 50 to 250 °C for 1 to 30 hours. The above-mentioned catalyst for hydrogenolysis may be supported on a carrier, such as carbon (e.g., activated carbon), alumina, silica, diatomaceous earth, titanium oxide, etc. in a proportion of 0.1 to 20 % by weight of the carrier. Of the above catalysts, preferred is a palladium catalyst, more preferably a palladium catalyst having a pH of 5 to 8. The catalyst is preferably freed of water in advance. The reaction may be performed in the presence or absence of an inert solvent, such as decane, octane, isooctane, heptane, hexane or cyclohexane. The starting materials of the acetal or ketal, i.e., glycerin or carbonyl compound (4) may be added to the reaction system. A trace amount of an acidic substance, such as phosphoric acid, may also be added to the reaction system. The reaction may be effected either in a closed system or in a hydrogen flow.

Isolation of compounds (1), (2), and (3) from the resulting reaction mixture can be carried out by filtering the reaction mixture to remove the catalyst and subjecting the mother liquor to general purification means, such as distillation for solvent removal, washing, recrystallization, distillation, and chromatography, either singly or in combination thereof.

Where the reaction mixture contains compound (2) in a large proportion, and much production of compound (1) is desired, it is preferable that the reaction mixture, after being filtered and evaporated, is hydrolyzed in a mixed acidic aqueous solution consisting of a sufficient amount of an acid, e.g., 0.1 to 1 N hydrochloric acid, and ethanol to convert compound (2) to compounds (1) and (4) and then subjected to recovering procedure to isolate compound (1) in an increased yield.

Where production of compound (2) is desired, it is preferable to add a large amount of the starting carbonyl compound (4) to the hydrogenation system and to stop the hydrogenation reaction halfway.

Where production of compound (3) is desired, it is preferable to add a large amount of the starting carbonyl compound (4) to the hydrogenation system and to completely perform hydrogenation.

Compounds (1A), (2A), and (3A) have low melting points and many of them are liquid at room temperature in spite of their large molecular weight because the alkyl moiety thereof is branched at the α-position. These compounds have satisfactory physical properties and good utility as lubricants or polar oils. They also have mutual action with water and are useful as emulsifying agents or moisturizing agents.

According to the process of the present invention, glycerin derivatives can be synthesized with high yield at low cost from economically and stably available starting materials (i.e., oleochemicals, aldehydes, ketones, glycerin). Unlike general etherification attended by by-production of sodium chloride, the process of the present invention attains satisfactory productivity without producing waste, thus satisfying the recent demand for environmental conservation.

The etherification process of the present invention does not use such a starting material as contains organic chlorine, organic bromine or organic iodine, such as epichlorohydrin or an alkyl halide, the resulting glycerin derivatives are completely freed of organic chlorine, organic bromine and organic iodine and excellent in stability. While conventional glyceryl ethers obtained by hydrolysis of glycidyl ethers have possibility of containing a residual epoxy group, such a fear is not at all entertained in the present invention.

The present invention will now be illustrated in greater detail with reference to Examples, but the present invention should not be construed as being limited thereto. In Examples, all the parts, percents, and ratios are given by weight unless otherwise noted.

## EXAMPLE 1

### Synthesis of 3-Hexyloxy-1,2-propanediol (1a)

(1) 2-Pentyl-1,3-dioxolane-4-methanol (6a) and 2-pentyl-1,3-dioxan-5-ol (5a):

In a 3 ℓ reaction vessel equipped with a thermometer, a reflux condenser, a Dean-Stark trap, a calcium chloride cylinder, and a stirrer were charged 400 g (3.994 mol) of caproaldehyde, 404.5 g (4.392 mol) of glycerin, 3.80 g (0.02 mol) of p-toluenesulfonic acid monohydrate, and 400 mℓ of hexane. The temperature was raised with stirring, and the mixture was allowed to react at 69 to 77 °C for 4 hours while removing a theoretical amount of water. After completion of the reaction, the reaction mixture was cooled to 60 °C, and 4.24 g (0.04 mol) of sodium carbonate was added thereto for neutralization, followed by stirring at 60 °C for 30 minutes. The temperature was gradually raised up to 130 °C to recover 350 mℓ of hexane and to obtain 774.9 g of a mixture of crude compounds (5a) and (6a).

The mixture was filtered and distilled under reduced pressure through a Vigreaux column (20 cm x 1.5 cm in diameter) to obtain 668.04 g (yield: 96.0 %) of a mixture of compounds (5a) and (6a).

Boiling Point: 87-102 °C/0.3 mmHg

Purity by gas chromatography (hereinafter referred to as GC purity): 94.1 %

Hydroxyl Value: 372.0 (theoretical value: 322.0)

(2) 3-Hexyloxy-1,2-propanediol (1a):

In a 0.5 ℓ autoclave were charged 200 g (1.148 mol) of compounds (5a) and (6a) prepared in (1) above and 4 g (2 %) of 5 % Pd/C (a dry preparation obtained by drying a 50 % hydrous 5 % Pd-on-carbon powder, "E-type" (pH = 6.9) produced by N.E. Chemcat Corporation, in a vacuum pump at room temperature for 1 day). The mixture was heated with stirring under a hydrogen pressure of 10 kg/cm$^2$, and allowed to react at 190 °C under a hydrogen pressure of 20 kg/cm$^2$ for 3 hours and then 200 kg/cm$^2$ for 4 hours. After completion of the reaction, the reaction mixture was filtered through a membrane filter (made of PTFE; pore size: 0.2 μm) under pressure to obtain 188.33 g (crude yield: 93.1 %) of a mixture of crude compound (1a) and 1,3-bishexyloxy-2-propanol (3a) (GC purity: 90 %). (1a)/(3a) = 7/3 (weight ratio).

To the mixture were added 200 mℓ of water, 200 mℓ of hexane, and 200 mℓ of methanol, and the mixture was shaken. The lower layer was further extracted with 200 mℓ of hexane. The hexane extract (lower layer) was furthermore extracted three times with ethyl acetate (200 mℓ, 100 mℓ and 100 mℓ). The extract was evaporated to obtain 73.29 g of crude compound (1a), which was distilled under reduced pressure through a Vigreaux column (20 cm x 1.5 cm in diameter) to obtain 55.7 g of compound (1a).

Boiling Point: 116-118 °C/1.5 mmHg

GC Purity: 95.1 %
Hydroxyl Value: 637.2 (theoretical value: 636.7)

EXAMPLE 2

Synthesis of 3-Hexyloxy-1,2-propanediol (1a)

In a 0.5 ℓ autoclave were added 200 g (1.148 mol) of the (5a)/(6a) mixture obtained in Example 1-(1) and 4 g (2 %) of 5 % Pd/C (a dry preparation prepared by drying a 50 % hydrous preparation of a 5 % Pd-on-carbon powder, "E-type" (pH = 6.9) produced by N. E. Chemcat Corporation, in a vacuum pump at room temperature for 1 day). The mixture was heated with stirring under a hydrogen pressure of 10 kg/cm$^2$, and allowed to react at 190 °C for 1 hour under a hydrogen pressure of 20 kg/cm$^2$ to obtain a mixture composed of 49 % of compound (1a) and 51 % of 2-pentyl-4-hexyloxymethyl-1,3-dioxolane. The reaction rate was 30 %, and the selectivity of hydrogenation was 90 %.

EXAMPLE 3

Synthesis of 3-Heptyloxy-1,2-propanediol (1b)

(1) (2)-Heptyl-1,3-dioxolane-4-methanol (6b) and 2-heptyl-1,3-dioxan-5-ol (5b):

In a 3 ℓ reaction vessel equipped with a thermometer, a reflux condenser, a Dean-Stark trap, a calcium chloride cylinder, and a stirrer were charged 400 g (3.503 mol) of heptaldehyde, 354.84 g (3.853 mol) of glycerin, 3.33 g (0.0175 mol) of p-toluenesulfonic acid monohydrate, 200 mℓ of toluene, and 200 mℓ of hexane. The temperature was raised with stirring, and the mixture was allowed to react at 82 to 94 °C for 5 hours while removing a theoretical amount of water. After completion of the reaction, the reaction mixture was cooled to 60 °C, and 3.71 g (0.035 mol) of sodium carbonate was added thereto for neutralization, followed by stirring at 60 °C for 30 minutes. To the reaction mixture was added 100 mℓ of water, followed by stirring at 60 °C for 30 minutes, and allowed to stand for liquid-liquid separation. The upper layer was washed with two 100 mℓ portions of a saturated sodium chloride aqueous solution and evaporated to obtain 636.8 g of a mixture of crude compound (5b) and crude compound (6b). The mixture was distilled under reduced pressure through a Vigreaux column (20 cm x 1.5 cm in diameter) to obtain 591.21 g (yield: 89.6 %) of a mixture of compounds (5b) and (6b).
Boiling Point: 87-102 °C/0.4 mmHg
GC Purity: 99.8 %
Hydroxyl value: 303.2 (theoretical value: 298.0)

(2) 3-Heptyloxy-1,2-propanediol (1b):

In a 0.5 ℓ autoclave were charged 100 g (0.531 mol) of compounds (5b) and (6b) prepared in (1) above and 2 g (2 %) of 5 % Pd/C (a dry preparation prepared by drying a 50 % hydrous preparation of a 5 % Pd-on-carbon powder, "E-type" (pH = 6.9) produced by N. E. Chemcat Corporation, in a vacuum pump at room temperature for 1 day). The mixture was heated with stirring under a hydrogen pressure of 10 kg/cm$^2$, and allowed to react at 190 °C under a hydrogen pressure of 20 kg/cm$^2$ for 6 hours and then at the same temperature under a hydrogen pressure of 200 kg/$^2$ for 4 hours. After completion of the reaction, the reaction mixture was filtered through a membrane filter (made of PTFE; pore size: 0.2 µm) under pressure to obtain 88.91 g (crude yield: 88 %) of a mixture of crude compound (1b) and 1,3-bisheptyloxy-2-propanol (3b) (GC purity: 90 %). (1b)/(3b) = 7/3 (weight ratio).

To the mixture were added 100 mℓ of water, 100 mℓ of hexane, and 100 mℓ of methanol, and the mixture was shaken. The lower layer was set aside, and the upper layer was further extracted 9 times with a mixture of 50 mℓ of water and 50 mℓ of methanol. The lower layers were combined and further extracted twice with 200 mℓ and 100 mℓ portions of ethyl acetate. The ethyl acetate extract was evaporated and distilled under reduced pressure through a Vigreaux column (20 cm x 1.5 cm in diameter) to obtain 36.46 g of compound (1b).
Boiling Point: 115-117 °C/0.3 mmHg
GC Purity: 98.3 %
Hydroxyl Value: 598.4 (theoretical value: 589.7)

EXAMPLE 4

Synthesis of 3-Isopropoxy-1,2-propanediol (1c)

In a 0.5 ℓ autoclave were added 136 g (1.029 mol) of solketal and 2.7 g (2 %) of 5 % Pd/C (a dry preparation prepared by drying a 50 % preparation of a 5 % Pd-on-carbon powder, "E-type" (pH = 6.24) produced by N. E. Chemcat Corporation, in a vacuum pump at room temperature for 1 day). The mixture was heated with stirring under a hydrogen pressure of 10 $kg/cm^2$, and allowed to react at 190 °C under a hydrogen pressure of 70 $kg/cm^2$ for 4 hours and then at the same temperature under a hydrogen pressure of 200 $kg/cm^2$ for 4 hours. After completion of the reaction, the reaction mixture was dissolved in isopropyl alcohol and filtered through 13.6 g of Kyowaad 600s under reduced pressure. The filtrate was evaporated to obtain 129.63 g (crude yield: 93.9 %) of a mixture of crude compound (1c) and 1,3-bisisopropoxy-2-propanol (3c) (GC purity: 91.6 %). (1c)/(3c) = 63/37 (weight ratio).

A 129.32 g portion of the mixture was distilled under reduced pressure through a Vigreaux column (20 cm x 1.5 cm in diameter) to obtain 58.13 g of compound (1c).

Boiling Point: 67-69 °C/0.5 mmHg
GC Purity: 99.9 %
Hydroxyl Value: 824.9 (theoretical value: 836.37)

EXAMPLE 5

Synthesis of 3-(1-Methylpropoxy)-1,2-propanediol (1d)

(1) 2-Methyl-2-ethyl-1,3-dioxolane-4-methanol (6d):

In a 3 ℓ reaction vessel equipped with a thermometer, a reflux condenser, a Dean-Stark trap, a calcium chloride cylinder, and a stirrer were charged 400 g (5.547 mol) of methyl ethyl ketone, 561.91 g (6.102 mol) of glycerin, 21.10 g (0.1109 mol) of p-toluenesulfonic acid monohydrate, and 400 mℓ of hexane. The temperature was raised with stirring, and the mixture was allowed to react at 67 to 74 °C for 30 hours while removing a theoretical amount of water. After completion of the reaction, the reaction mixture was cooled to 60 °C, and 23.51 g (0.2218 mol) of sodium carbonate was added thereto for neutralization, followed by stirring at 60 °C for 30 minutes. The reaction mixture was gradually heated up to 120 °C to recover 400 mℓ of hexane. The residue was filtered to obtain 894.74 g of crude compound (6d), which was then distilled under reduced pressure through a Vigreaux column (20 cm x 1.5 cm in diameter) to obtain 725.14 g (yield: 89.4 %) of compound (6d).

Boiling point: 101-104 °C/17 mmHg
GC Purity: 99.1 %
Hydroxyl value: 381.8 (theoretical value: 383.8)

(2) 3-(1-Methylpropoxy)-1,2-propanediol (1d):

In a 0.5 ℓ autoclave were charged 150 g (1.026 mol) of compound (6d) prepared in (1) above and 3 g (2 %) of 5 % Pd/C (a dry preparation prepared by drying a 50 % hydrous preparation of a 5 % Pd-on-carbon powder, "E-type" (pH = 6.9) produced by N. E. Chemcat Corporation, in a vacuum pump at room temperature for 1 day). The mixture was heated with stirring under a hydrogen pressure of 10 $kg/cm^2$, and allowed to react at 190 °C under a hydrogen pressure of 20 $kg/cm^2$ for 6 hours and then at the same temperature under a hydrogen pressure of 200 $kg/^2$ for 3 hours. After completion of the reaction, the reaction mixture was filtered through a membrane filter (made of PTFE; pore size: 0.2 µm) under pressure to obtain 141.76 g (crude yield: 93.2 %) of a mixture of crude compound (1d) and 1,3-bis(1-methylpropoxy)-2-propanol (3d) (GC purity: 86.3 %). (1d)/(3d) = 7/3 (weight ratio).

The mixture was distilled under reduced pressure through a Vigreaux column (10 cm x 1.5 cm in diameter) to obtain 6.53 g of compound (1d).

Boiling Point: 69-70 °C/0.3 mmHg
GC Purity: 99.9 %
Hydroxyl Value: 761.9 (theoretical value: 757.2)

EXAMPLE 6

Synthesis of 3-(1,3-Dimethylbutoxy)-1,2-propanediol (1e)

(1) 2-Methyl-2-isobutyl-1,3-dioxolane-4-methanol (6e):

In a 2 ℓ reaction vessel equipped with a thermometer, a reflux condenser, a Dean-Stark trap, a calcium chloride cylinder, and a stirrer were charged 790 g (7.887 mol) of methyl isobutyl ketone, 484.2 g (5.258 mol) of glycerin, 20.0 g (0.105 mol) of p-toluenesulfonic acid monohydrate, and 400 mℓ of hexane. The temperature was raised with stirring, and the mixture was allowed to react at 85 to 90 °C for 105 hours while removing a theoretical amount of water. After completion of the reaction, the reaction mixture was cooled to 40 °C, and 22.29 g (0.210 mol) of sodium carbonate was added thereto for neutralization, followed by stirring at 50 °C for 1 hour. To the mixture was added 300 mℓ of water, and the mixture was stirred at room temperature for 30 minutes, followed by allowing to stand for liquid-liquid separation. After removin the lower layer, the upper layer was washed twice with a saturated sodium chloride aqueous solution (250 mℓ/200 mℓ), dried over sodium sulfate, and evaporated to obtain 1050.1 g (crude yield: 93 %) of crude product (6e) (purity: 81.1 %). The crude product was distilled under reduced pressure to obtain 824.5 g (yield: 90.0 %) of compound (6e).

Boiling point: 76 °C/1 mmHg
GC Purity: 97.3 %
Hydroxyl value: 314.1 (theoretical value: 322.03)

(2) 3-(1,3-Dimethylbutoxy)-1,2-propanediol (1e):

In a 0.5 ℓ autoclave were charged 290 g (1.664 mol) of compound (6e) prepared in (1) above and 2.9 g (1 %) of 5 % Pd/C (a dry preparation prepared by drying a 50 % hydrous preparation a 5 % Pd-on-carbon powder, "E-type" (pH = 6.24) produced by N. E. Chemcat Corporation, in a vacuum pump at room temperature for 1 day). The mixture was heated with stirring under a hydrogen pressure of 10 kg/$cm^2$, and allowed to react at 190 °C under a hydrogen pressure of 70 kg/$cm^2$ for 11 hours and then at the same temperature under a hydrogen pressure of 200 kg/$^2$ for 10 hours. After completion of the reaction, the reaction mixture was filtered through 27 g of Kyowaad 600s to obtain 288.5 g (crude yield: 98.3 %) of a mixture of crude compound (1e) and 1,3-bis(1,3-dimethylbutoxy)-2-propanol (3e) (GC purity: 90.6 %). (1e)/(3e) = 72/28 (weight ratio).

A 192.38 g portion of the mixture was purified by silica gel column chromatography (eluent: chloroform/methanol).

The second fraction was distilled under reduced pressure through a Vigreaux column (12 cm x 1.5 cm in diameter) to obtain 116.87 g of compound (1e).

Boiling Point: 89-90 °C/0.5 mmHg
GC Purity: 100 %
Hydroxyl Value: 632.0 (theoretical value: 636.69)

EXAMPLE 7

Synthesis of 3-(1,3-Dimethylbutoxy)-1,2-propanediol (1e)

In a 0.5 ℓ autoclave were added 100 g (0.574 mol) of compound (6e) obtained in Example 6-(1) and 1.0 g (1 %) of 5 % Pd/C (a dry preparation prepared by drying a 50 % hydrous preparation of a 5 % Pd-on-carbon powder, "E-type" (pH = 6.24) produced by N. E. Chemcat Corporation, in a vacuum pump at room temperature for 1 day). The mixture was heated with stirring under a hydrogen pressure of 10 kg/$cm^2$, and allowed to react at 190 °C for 4 hour under a hydrogen pressure of 70 kg/$cm^2$ to obtain a mixture composed of 42 % of compound (1e), 7 % of 1,3-bis-(1,3-dimethylbutoxy)-2-propanol, and 51 % of 2-methyl-2-isobutyl-4-(1,3-dimethylbutoxy)-1,3-dioxolane.The reaction rate was 60 %, and the selectivity of hydrogenation was 90 %. Compound (1e) was isolated from the mixture by silica gel column chromatography in the same manner as in Example 6.

EXAMPLE 8

Synthesis of 3-(1,3-Dimethylbutoxy)-1,2-propanediol (1e)

In a 0.5 ℓ autoclave were added 50 g (0.287 mol) of compound (6e) obtained in Example 6-(1) and 1.0 g (1 %) of 5 % Pd/C (a dry preparation prepared by drying a 50 % hydrous preparation of a 5 % Pd-on-carbon powder, "E-type" (pH = 6.24) produced by N. E. Chemcat Corporation, in a vacuum pump at room temperature for 1 day). The mixture was heated with stirring under a hydrogen pressure of 10 kg/cm$^2$ and allowed to react at 190 °C under a hydrogen pressure of 20 kg/cm$^2$ for 6 hours to obtain a mixture composed of 72 % of compound (1e), 26 % of 1,3-bis-(1,3-dimethylbutoxy)-2-propanol, and 2 % of 2-methyl-2-isobutyl-4-(1,3-dimethylbutoxy)-1,3-dioxolane. The reaction rate was 99 %, and the selectivity of hydrogenation was 90 %. Compound (1e) was isolated from the mixture by silica gel column chromatography in the same manner as in Example 6.

EXAMPLE 9

Synthesis of 3-(1,3-Dimethylbutoxy)-1,2-propanediol (1e)

In a 0.5 ℓ autoclave were added 30 g (0.172 mol) of compound (6e) obtained in Example 6-(1) and 1.5 g (5 %) of 5 % Pd/C (a dry preparation prepared by drying a 50 % hydrous preparation of a 5 % Pd-on-carbon powder, "E-type" (pH = 6.24) produced by N. E. Chemcat Corporation, in a vacuum pump at room temperature for 1 day). The mixture was heated with stirring under a hydrogen pressure of 20 kg/cm$^2$ while introducing hydrogen at a flow rate of 5 ℓ/min, and allowed to react at 190 °C under a hydrogen pressure of 20 kg/cm$^2$ for 6 hours while introducing hydrogen at a flow rate of 5 ℓ/min to obtain a mixture composed of 74 % of compound (1e) and 26 % of 1,3-bis-(1,3-dimethylbutoxy)-2-propanol. The reaction rate was 100 %, and the selectivity of hydrogenation was 90 %. Compound (1e) was isolated from the mixture by silica gel column chromatography in the same manner as in Example 6.

EXAMPLE 10

Synthesis of 3-(1,3-Dimethylbutoxy)-1,2-propanediol (1e)

In a 0.5 ℓ autoclave were added 30 g (0.172 mol) of compound (6e) obtained in Example 6-(1), 0.6 g (2 %) of 5 % Pd/C (a dry preparation prepared by drying a 50 % preparation of a 5 % Pd-on-carbon powder, "E-type" (pH = 6.24) produced by N. E. Chemcat Corporation, in a vacuum pump at room temperature for 1 day), and 6 g (0.0651 mol) of glycerin. The mixture was heated with stirring under a hydrogen pressure of 20 kg/cm$^2$ while introducing hydrogen at a flow rate of 2.5 ℓ/min, and allowed to react at 190 °C for 6 hours under a hydrogen pressure of 20 kg/cm$^2$ while introducing hydrogen at a flow rate of 2.5 ℓ/min to obtain a mixture composed of 83 % of compound (1e) and 17 % of 1,3-bis-(1,3-dimethylbutoxy)-2-propanol. The reaction rate was 100 %, and the selectivity of hydrogenation was 90 %. Compound (1e) was isolated from the mixture by silica gel column chromatography in the same manner as in Example 6.

EXAMPLE 11

Synthesis of 3-(1,3-Dimethylbutoxy)-1,2-propanediol (1e)

(1) 2-Methyl-2-isobutyl-1,3-dioxolane-4-methanol (6e):

In a 2 ℓ reaction vessel equipped with a thermometer, a reflux condenser, a Dean-Stark trap, a calcium chloride cylinder, and a stirrer were charged 326.3 g (3.258 mol) of methyl isobutyl ketone, 200 g (2.172 mol) of glycerin, 8.3 g (0.436 mol) of p-toluenesulfonic acid monohydrate, 200 mℓ of toluene, and 200 mℓ of hexane. The temperature was raised with stirring, and the mixture was allowed to react at 90° to 93 °C for 12 hours while removing a theoretical amount of water. After completion of the reaction, the reaction mixture was cooled to 60 °C, and 9.24 g (0.0872 mol) of sodium carbonate was added thereto for neutralization, followed by stirring at 60 °C for 30 minutes. The reaction mixture was gradually heated up to 150 °C to recover hexane and toluene. The residue was filtered, and the filtrate was distilled under reduced pressure through a Vigreaux column (20 cm x 1.5 cm in diameter) to obtain 341 g (yield: 90.1 %) of compound (6e).

Boiling point: 65-68 °C/0.5 mmHg
GC Purity: 98.9 %
Hydroxyl value: 306.1 (theoretical value: 322.03)

(2) 3-(1,3-Dimethylbutoxy)-1,2-propanediol (1e):

In a 0.5 ℓ autoclave were charged 30 g (0.172 mol) of compound (6e) prepared in (1) above, 1.5 g (5 %) of 5 % Pd/C (a dry preparation prepared by drying a 50 % hydrous preparation of a 5 % Pd-on-carbon powder, "K-type" (pH = 8.2) produced by N. E. Chemcat Corporation, in a vacuum pump at room temperature for 1 day), and 6.88 g (0.0747 mol) of glycerin. The mixture was heated with stirring under a hydrogen pressure of 10 kg/cm$^2$ while introducing hydrogen at a flow rate of 2.5 ℓ/min, and allowed to react at 190 °C under a hydrogen pressure of 20 kg/cm$^2$ for 6 hours while introducing hydrogen at a flow rate of 2.5 ℓ/min to obtain a mixture of 86 % of compound (1e) and 14 % of 1,3-bis-(1,3-dimethylbutoxy)-2-propanol. The reaction rate was 100 %, and the selectivity of hydrogenation was 90 %. Compound (1e) was isolated from the mixture by silica gel column chromatography in the same manner as in Example 6.

EXAMPLE 12

Synthesis of 3-(1-Heptadecyloctadecyloxy)-1,2-propanediol (1f)

(1) 2,2-Diheptadecyl-1,3-dioxolane-4-methanol (6f):

In a 500 mℓ reaction vessel equipped with a thermometer, a reflux condenser, a Dean-Stark trap, a calcium chloride cylinder, and a stirrer were charged 200 g (0.395 mol) of diheptadecyl ketone, 36.34 g (0.395 mol) of glycerin, 0.38 g (0.002 mol) of p-toluenesulfonic acid monohydrate, and 50 mℓ of hexane. The temperature was raised with stirring, and the mixture was allowed to react at 100 °C for 136 hours. A theoretical amount of water and also hexane were removed by distillation to obtain 225.26 g of a reaction mixture containing compound (6f). The reaction rate was 85.4 % as determined by gas chromatography. A 124.31 g portion of the reaction mixture was dissolved in 600 mℓ of hexane, and the solution was neutralized with 1.2 g of sodium carbonate, followed by stirring at 50 °C for 1 hour. Insoluble matters were removed by filtration at 50 °C, and the filtrate was cooled to room temperature to recover 7.87 g of the thus precipitated unreacted diheptadecyl ketone by filtration. The filtrate was cooled to -20 °C, and the precipitate was collected by filtration and dried in a desiccator under reduced pressure to obtain 95.23 g (yield: 75.2 %) of compound (6f) (melting point: 37-39 °C).

(2) 3-(1-Heptadecyloctadecyloxy)-1,2-propanediol (1f):

In a 0.5 ℓ autoclave were charged 50 g (0.086 mol) of compound (6f) prepared in (1) above, 100 mℓ of hexane, and 0.9 g (1.8 %) of 5 % Pd/C (a dry preparation prepared by drying a 50 % hydrous preparation of a 5 % Pd-on-carbon powder, "E-type" (pH = 6.24) produced by N. E. Chemcat Corporation, in a vacuum pump at room temperature for 1 day). The mixture was heated with stirring under a hydrogen pressure of 10 kg/cm$^2$ and allowed to react at 190 °C under a hydrogen pressure of 175 to 250 kg/cm$^2$ for 2 hours. After completion of the reaction, the reaction mixture was filtered under reduced pressure by using Celite 545 as a filter aid to remove the catalyst. The filtrate was evaporated to obtain 43.33 g (crude yield: 86.4 %) of a mixture containing compound (1f) (melting point: 36-38 °C). A 5 g portion of the reaction mixture was dissolved in 20 mℓ of chloroform, and the solution was passed through a column packed with 150 g of Wako Gel C-200 (produced by Wako Pure Chemical Industries, Ltd.) and eluted first with chloroform to recover the by-products and the unreacted starting material and then with a 98/2 mixture of chloroform and methanol to recover 0.95 g (yield: 16.4 %) of compound (1f).

Melting Point: 55-56 °C
GC Purity: 98.6 %

| | |
|---|---|
| IR (KBr, cm$^{-1}$): | 3416 (O-H stretching), 2920, 2848 (C-H stretching), 1468, 1378 (C-H deformation), 1116 (C-O-C stretching), 1092, 1050 (C-O stretching), 720 ($CH_2$ rocking) |
| NMR ($CDC\ell_3$, $\delta$ ppm): | 0.88 (6H, triplet, J = 6.5Hz, -C$\underline{H}_3$), 1.00-1.70 (64H, broad, -C$\underline{H}_2$-), 1.30-1.70 (2H, broad, -O$\underline{H}$), 3.28 (1H, quintet, J = 5.0Hz, >C$\underline{H}$-O-), 3.40-3.90 (5H, multiplet, -OC$\underline{H}_2$C$\underline{H}$(OH)C$\underline{H}_2$OH) |

EXAMPLE 13

Synthesis of 3-(1-Heptadecyloctadecyloxy)-1,2-propanediol (1f)

(1) 2,2-Diheptadecyl-1,3-dioxolane-4-methanol (6f):

In a 1 ℓ reaction vessel equipped with a thermometer, a reflux condenser, a Dean-Stark trap, a calcium chloride cylinder, and a stirrer were charged 500 g (0.986 mol) of diheptadecyl ketone, 99.92 g (1.085 mol) of glycerin, 3.75 g (0.0197 mol) of p-toluenesulfonic acid monohydrate, and 160 mℓ of hexane. The temperature was raised with stirring, and the mixture was allowed to react at 100 °C for 190 hours. After a theoretical amount of water was removed, the reaction mixture was cooled to 60 °C. To the reaction mixture was added 20.88 g (0.197 mol) of sodium carbonate, and the mixture was stirred at 60 °C for 1 hour for neutralization, followed by filtration to remove 35.29 g of insoluble matter. The filtrate was cooled to room temperature to recover 36.32 g of the unreacted diheptadecyl ketone by filtration. The filtrate was cooled to -20 °C, and the precipitate was collected by filtration and dried under reduced pressure to obtain 487.28 g (yield: 85.1 %) of compound (6f) (melting point: 37-39 °C).

(2) 3-(1-Heptadecyloctadecyloxy)-1,2-propanediol (1f):

In a 1 ℓ autoclave were charged 200 g (0.344 mol) of compound (6f) prepared in (1) above, 400 mℓ of hexane, and 3.8 g (1.9 %) of 5 % Pd/C (a dry preparation prepared by drying a 50 % hydrous preparation of a 5 % Pd-on-carbon powder, "E-type" (pH = 6.24) produced by N. E Chemcat Corporation, in a vacuum pump at room temperature for 1 day). The mixture was heated with stirring under a hydrogen pressure of 10 $kg/cm^2$ and allowed to react at 190 °C under a hydrogen pressure of 200 $kg/cm^2$ for 15 hours.

After completion of the reaction, the reaction mixture was filtered under reduced pressure through 20 g of Kyowaad 600s as a filter aid to separate 36.17 g of a solid (the catalyst and diheptadecyl ketone). The filtrate was evaporated to obtain a solid weighing 151.11 g. A 150 g portion of the solid was dissolved in 500 mℓ of ethanol, and the solution was charged in a 1 ℓ four-necked flask equipped with a thermometer, a reflux condenser, and a stirring rod together with 15 g of 36 % HCℓ and 135 g of water. The mixture was refluxed at 80 °C for 24 hours while stirring, followed by cooling. The thus formed precipitate (135.18 g) was collected by filtration and crystallized from hexane to recover 77.72 g of diheptadecyl ketone by filtration. The crystallization mother liquor was concentrated to obtain 57.08 g of a mixture (A) of compound (1f) and 1,3-bis-(1-heptadecyloctadecyloxy)-2-propanol.

The mother liquor of the reaction mixture (after removal of the insoluble matter) was evaporated to remove ethanol, and the residue was re-dissolved in 200 mℓ of hexane, washed with water and then with a saturated sodium hydrogencarbonate aqueous solution, dried over sodium sulfate, and evaporated to obtain 5.85 g of a mixture (B) of compound (1f) and 1,3-bis(1-heptadecyloctadecyloxy)-2-propanol. Mixtures (A) and (B) were combined (amounting to 62.93 g) and dissolved in 100 mℓ of chloroform, and the solution was passed through a column packed with 400 g of Wako Gel C-200 and eluted first with chloroform to separate the by-product and then with a 98/2 mixture of chloroform and methanol to recover 39.37 g of compound (1f). The yield was 62 %, with the recovered diheptadecyl ketone being taken into consideration.

Melting Point: 55-56 °C
GC Purity: 99.3 %
HPLC Purity: 98.4 %
Hydroxyl Value: 190.6 (theoretical value: 192.47)

EXAMPLE 14

Synthesis of 3-(1-Pentylhexyloxy)-1,2-propanediol (1g)

(1) 2,2-Dipentyl-1,3-dioxolane-4-methanol (6g):

In a 1 ℓ reaction vessel equipped with a thermometer, a reflux condenser, a Dean-Stark trap, a calcium chloride cylinder, and a stirrer were charged 400 g (2.349 mol) of dipentyl ketone, 259.59 g (2.819 mol) of glycerin, 4.47 g (0.0235 mol) of p-toluenesulfonic acid monohydrate, and 120 mℓ of hexane. The temperature was raised with stirring, and the mixture was allowed to react at 100 °C for 126 hours. After a theoretical amount of water was removed, the reaction mixture was cooled to 60 °C, neutralized with 24.91 g (0.235 mol) of sodium carbonate, and stirred at 60 °C for 1 hour. To the mixture was added 100 mℓ of

water, followed by stirring at 50 to 60 °C for 15 minutes. The mixture was allowed to stand, and the aqueous layer was removed. The organic layer was washed with 100 mℓ of water and evaporated to obtain 563.05 g of crude compound (6g) (GC purity: 87.0 %). A 561.20 g portion of the crude product was distilled to obtain 412.86 g (yield: 72.1 %) of compound (6g).

Boiling Point: 128-130 °C/1 mmHg
GC Purity: 97.7 %
Hydroxyl Value: 237.4 (theoretical value: 229.6)

(2) 3-(1-Pentylhexyloxy)-1,2-propanediol (1g):

In a 0.5 ℓ autoclave were charged 50 g (0.205 mol) of compound (6g) prepared in (1) above, 200 mℓ of hexane, and 1 g (2 %) of 5 % Pd/C (a dry preparation prepared by drying a 50 % hydrous preparation of a 5 % Pd-on-carbon powder, "E-type" (pH = 6.24) produced by N. E. Chemcat Corporation, in a vacuum pump at room temperature for 1 day). The mixture was heated with stirring under a hydrogen pressure of 10 kg/cm$^2$ and allowed to react at 130 °C under a hydrogen pressure of 200 kg/cm$^2$ for 6 hours and then at 190 °C under the same hydrogen pressure for 3 hours. During the reaction, the reaction mixture was occasionally sampled.

After completion of the reaction, the reaction mixture was filtered under reduced pressure through Kyowaad 600s as a filter aid to remove the catalyst, and the filtrate was evaporated to recover 40.2 g of crude compound (1g). The resulting crude compound (1g), 200 mℓ of ethanol, 1 g of 36 % HCℓ, and 100 g of water were charged in a 500 mℓ reaction vessel equipped with a thermometer, a reflux condenser, and a stirring rod. The mixture was heat-refluxed while stirring at 80 °C for 3 hours. After the completion of the reaction, the reaction mixture was cooled to room temperature, ethanol was removed by evaporation, and 200 mℓ of hexane was added to the residue. The solution was washed with 100 mℓ of water and then with 100 mℓ of a saturated sodium hydrogencarbonate aqueous solution, dried over sodium sulfate, and evaporated. The residual oily substance weighing 38 g was distilled under reduced pressure to obtain 21.79 g (yield: 57.3 %) of compound (1g).

Boiling Point: 143-145 °C/1 mmHg
GC Purity: 97.1 %
Hydroxyl Value: 442.0 (theoretical value: 455.44)

| | |
|---|---|
| IR (neat, cm$^{-1}$): | 3396 (O-H stretching), 2928, 2860 (C-H stretching), 1462, 1380 (C-H deformation), 1098 (C-O-C stretching), 1064, 1050, 930 (C-O stretching), 724 ($CH_2$, rocking) |
| NMR ($CDCℓ_3$, δ ppm): | 0.88 (6H, triplet, J = 6.7Hz, -C$\underline{H}_3$), 1.00-1.70 (16H, broad, -C$\underline{H}_2$), 2.20-2.70 (2H, broad, -O$\underline{H}$), 3.28 (1H, quintet, J = 5.0Hz, >C$\underline{H}$-O-), 3.40-4.00 (5H, multiplet, -O-C$\underline{H}_2$-C$\underline{H}$(OH)C$\underline{H}_2$OH) |

## EXAMPLE 15

### Synthesis of 3-(1-Heptyloctyloxy)-1,2-propanediol (1h)

(1) 2,2-Diheptyl-1,3-dioxolane-4-methanol (6h):

In a 300 mℓ reaction vessel equipped with a thermometer, a reflux condenser, a Dean-Stark trap, a calcium chloride cylinder, and a stirrer were charged 100 g (0.442 mol) of diheptyl ketone, 44.75 g (0.486 mol) of glycerin, 1.68 g (0.00832 mol) of p-toluenesulfonic acid monohydrate, and 30 mℓ of hexane. The temperature was raised with stirring, and the mixture was allowed to react at 100 °C for 78 hours. After a theoretical amount of water was distilled off, the reaction mixture was cooled to 60 °C, neutralized with 8.82 g (0.0832 mol) of sodium carbonate, and stirred at 60 °C for 1 hour. To the mixture was added 50 mℓ of water, followed by stirring at 60 °C for 1 hour. The mixture was allowed to stand, and the aqueous layer was removed. The organic layer was washed with three 50 mℓ portions of water, and the resulting organic layer was dried over sodium sulfate and evaporated to obtain 134.93 g of crude compound (6h) (GC purity: 90 %). Distillation of the crude product gave 101.65 g (yield: 76.5 %) of compound (6h).

Boiling Point: 152-153 °C/0.5 mmHg
GC Purity: 97.2 %
Hydroxyl Value: 194.9 (theoretical value: 186.72)

(2) 3-(1-Heptyloctyloxy)-1,2-propanediol (1h):

In a 0.5 ℓ autoclave were charged 60 g (0.200 mol) of compound (6h) prepared in (1) above and 1.2 g (2 %) of 5 % Pd/C (a dry preparation prepared by drying a 50 % hydrous preparation of a 5 % Pd-on-carbon powder, "E-type" (pH = 6.24) produced by N. E. Chemcat Corporation, in a vacuum pump at room temperature for 1 day). The mixture was heated with stirring under a hydrogen pressure of 10 $kg/cm^2$ and allowed to react at 130 °C under a hydrogen pressure of 70 $kg/cm^2$ for 5 hours and then at 190 °C under the same hydrogen pressure for 2 hours. During the reaction, the reaction mixture was occasionally sampled.

After completion of the reaction, the reaction mixture was filtered under reduced pressure through 6.0 g of Kyowaad 600s as a filter aid to remove the catalyst, and the filtrate was evaporated to recover 51.97 g of crude compound (1h). The resultant crude product, 200 mℓ of ethanol, 10 g of 36 % HCℓ, and 90 g of water were charged in a 500 mℓ reaction vessel equipped with a thermometer, a reflux condenser, and a stirring rod, and the mixture was heat-refluxed while stirring at 80 °C for 3 hours. After cooling to room temperature, ethanol was removed by evaporation, and 200 mℓ of hexane was added to the residue. The solution was washed successively with 100 mℓ of water and 100 mℓ of a saturated sodium hydrogencarbonate aqueous solution, dried over sodium sulfate, and evaporated. The residual oily substance weighing 50.57 g was distilled under reduced pressure to obtain 26.95 g (yield: 53.3 %) of compound (1h).

Boiling Point: 160-162 °C/0.4 mmHg
GC Purity: 96.8 %
Hydroxyl Value: 360.7 (theoretical value: 370.96)

| | |
|---|---|
| IR (neat, $cm^{-1}$): | 3412 (O-H stretching), 2924, 2856 (C-H stretching), 1462, 1378 (C-H deformation), 1104 (C-O-C stretching), 1062, 1050 (C-O stretching), 722 ($CH_2$, rocking) |
| NMR ($CDCℓ_3$, δ ppm): | 0.89 (6H, triplet, J = 6.4Hz, -C<u>H</u>$_3$), 1.08-1.70 (24H, broad, -C<u>H</u>$_2$), 2.20-2.60 (2H, broad, -O<u>H</u>), 3.27 (1H, quintet, J = 5.6Hz, >C<u>H</u>-O-), 3.37-4.00 (5H, multiplet, -O-C<u>H</u>$_2$-C<u>H</u>(OH)C<u>H</u>$_2$OH) |

<u>EXAMPLES 16 TO 19</u>

The alkyl glyceryl ethers shown in Table 1 below were synthesized in the same manner as in Example 15. Comparative compounds shown in Table 2 below were also synthesized for the sake of comparison of melting point.

TABLE 1

| $R^1$ and $R^2$ | Compound Designation | Yield of Ketal (%) | Yield of Hydro-genation (%) | GC Purity (%) | HPLC Purity (%) | Purification | Hydroxyl Value* | Physical Properties |
|---|---|---|---|---|---|---|---|---|
| $C_9H_{19}$ | Compound (1i) | 99 | 52 | 98.7 | 98.6 | column chro-matography | 313.3 (312.92) | liquid |
| $C_{11}H_{23}$ | Compound (1j) | 86 | 53 | 98.1 | 97.7 | column chro-matography | 265.2 (270.58) | solidify at around room temp. |
| $C_{13}H_{27}$ | Compound (1k) | 100 | 23 | 97.2 | 98.3 | column chro-matography | 229.4 (238.34) | m.p.= 36-38°C |
| $C_{15}H_{31}$ | Compound (1l) | 97 | 74 | 97.8 | 97.0 | column chro-matography | 207.8 (212.96) | m.p.= 46-47°C |

Note: *: The values in the parentheses are theoretical ones.

TABLE 2

$$\underset{\displaystyle ROCH_2CHCH_2OH}{\overset{\displaystyle OH}{|}}$$

| R | Comparative Compound | Physical Properties |
|---|---|---|
| $C_{18}H_{37}$ | batyl alcohol | m.p.=71-73°C |
| $C_{16}H_{33}$ | chimyl alcohol | m.p.=64°C |
| $C_{12}H_{25}$ | lauryl glyceryl ether | m.p.=40-42°C |

It is seen that the compounds of the present invention shown in Table 1 are substantially equal in melting point to the comparative straight-chain alkyl glyceryl ethers shown in Table 2, while the former compounds have about doubled alkyl chain length.

TEST EXAMPLE 1

The compound of the present invention or a comparative compound shown in Table 3 below (0.1 g) and 1.4 g of squalane were heated to melt. To the molten mixture was added 3.5 g of ion-exchanged water all at once at room temperature, followed by stirring in a homogenizer at 9000 rpm. The state of the resulting mixture was observed at room temperature. The results are shown in Table 3.

TEST EXAMPLE 2

Water was gradually added to each 1 g of the compound of the present invention or a comparative compound shown in Table 3 below while mixing by means of a spatula at room temperature until water separate from the mixture. The amount of water added until water separate from the mixture (% by weight) was referred to as the rate of water solubilization. A higher rate of water solubilization indicates a higher interaction between the compound and water, thus it can be used as an index of an emollient property and a moisture-keeping property of the compound. The results are also shown in Table 3.

TABLE 3

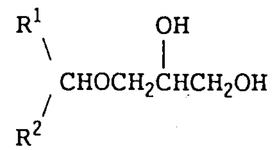

| $R^1$ | $R^2$ | Compound | State (room temperature) | Rate of Water Solubilization |
|---|---|---|---|---|
| $C_5H_{11}$ | $C_5H_{11}$ | compound (1g) | two-phase separation (O/W emulsion in the upper layer) | 10 % |
| $C_7H_{15}$ | $C_7H_{15}$ | compound (1h) | two-phase separation (O/W emulsion in the upper layer) | 10 % |
| $C_{13}H_{27}$ | $C_{13}H_{27}$ | compound (1k) | two-phase separation (O/W emulsion in the upper layer) | - |
| H | $C_{11}H_{23}$ | Comparative Compound (a) | two-phase separation (W/O emulsion) | - |
| H | $i\text{-}C_{17}H_{35}$ | Comparative Compound (b) | uniform emulsion (W/O emulsion) liquid crystal | 8 %* |

*: Through observation by a polarizing microscope, it was confirmed that compounds having a rate of water solubilization of from 8 to 28 % give reverse-hexagonal liquid crystals.

EXAMPLE 20

Synthesis of 2-Pentyl-4-hexyloxymethyl-1,3-dioxolane (2a)

(1) 2-Pentyl-1,3-dioxolane-4-methanol (6a) and 2-Pentyl-1,3-dioxan-5-ol (5a):

In a 3 ℓ reaction vessel equipped with a thermometer, a reflux condenser, a Dean-Stark trap, a calcium chloride cylinder, and a stirrer were charged 400 g (3.994 mol) of caproaldehyde, 404.5 g (4.392 mol) of glycerin, 3.80 g (0.02 mol) of p-toluenesulfonic acid monohydrate, and 400 mℓ of hexane. The temperature was raised with stirring, and the mixture was allowed to react at 69 to 77 °C for 4 hours while removing a theoretical amount of water. After completion of the reaction, the reaction mixture was cooled to 60 °C, and 4.24 g (0.04 mol) of sodium carbonate was added thereto for neutralization, followed by stirring at 60 °C for 30 minutes. The mixture was heated up to 130 °C to recover 350 mℓ of hexane and to obtain 774.9 g of crude compounds (5a) and (6a).

The crude product was distilled under reduced pressure through a Vigreaux column (20 cm x 1.5 cm in diameter) to obtain 668.04 g (yield: 96.0 %) of compounds (5a) and (6a).

Boiling Point: 87-102 °C/0.3 mmHg

GC Purity: 94.1 %

Hydroxyl Value: 372.0 (theoretical value: 322.0)

(2) 2-Pentyl-4-hexyloxymethyl-1,3-dioxolane (2a):

In a 0.5 ℓ autoclave were added 200 g (1.148 mol) of compounds (5a) and (6a) obtained in (1) above and 4 g (2 %) of 5 % Pd/C (a dry preparation prepared by drying a 50 % preparation of a 5 % Pd-on-carbon powder, "E-type" (pH = 6.9) produced by N. E. Chemcat Corporation, in a vacuum pump at room temperature for 1 day). The mixture was heated with stirring under a hydrogen pressure of 10 $kg/cm^2$ and allowed to react at 190 °C for 1 hour under a hydrogen pressure of 20 $kg/cm^2$ to obtain a mixture composed of 51 % of compound (2a) and 49 % of 3-hexyloxy-1,2-propanediol. The reaction rate was 30 %, and the selectivity of hydrogenation was 90 %. Compound (2a) was isolated from the mixture by column chromatography on alumina.

EXAMPLE 21

Synthesis of 2-Methyl-2-isobutyl-4-(1,3-dimethylbutoxy)-1,3-dioxolane (2e)

(1) 2-Methyl-2-isobutyl-1,3-dioxolane-4-methanol (6e):

In a 2 ℓ reaction vessel equipped with a thermometer, a reflux condenser, a Dean-Stark trap, a calcium chloride cylinder, and a stirrer were charged 790 g (7.887 mol) of methyl isobutyl ketone, 484.2 g (5.258 mol) of glycerin, 20.0 g (0.105 mol) of p-toluenesulfonic acid monohydrate, and 400 mℓ of hexane. The temperature was raised with stirring, and the mixture was allowed to react at 85 to 90 °C for 105 hours to remove a theoretical amount of water. After completion of the reaction, the reaction mixture was cooled to 40 °C, and 22.29 g (0.210 mol) of sodium carbonate was added thereto for neutralization, followed by stirring at 50 °C for 1 hour. To the mixture was added 300 mℓ of water, and the mixture was stirred at room temperature for 30 minutes and allowed to stand for liquid-liquid separation. The lower layer was removed, and the upper layer was washed twice with a saturated sodium chloride aqueous solution (250 mℓ/200 mℓ), dried over sodium sulfate, and evaporated to obtain 1050.1 g (crude yield: 93 %) of crude compound (6e) (purity: 81.1 %). The crude compound was distilled under reduced pressure to obtain 824.5 g (yield: 90.0 %) of compound (6e).

Boiling Point: 76 °C/1 mmHg

GC Purity: 97.3 %

Hydroxyl Value: 314.1 (theoretical value: 322.03)

(2) 2-Methyl-2-isobutyl-4-(1,3-dimethylbutoxy)-1,3-dioxolane (2e):

In a 0.5 ℓ autoclave were added 100 g (0.574 mol) of compounds (6e) obtained in (1) above and 1.0 g (1 %) of 5 % Pd/C (a dry preparation prepared by drying a 50 % preparation of a 5 % Pd-on-carbon powder, "E-type" (pH = 6.24) produced by N. E. Chemcat Corporation, in a vacuum pump at room

temperature for 1 day). The mixture was heated with stirring under a hydrogen pressure of 10 kg/$cm^2$ and allowed to react at 190 °C for 4 hours under a hydrogen pressure of 70 kg/$cm^2$ to obtain a mixture composed of 51 % of compound (2e), 42 % of 3-(1,3-dimethylbutoxy)-1,2-propanediol, and 7 % of 1,3-bis-(1,3-dimethylbutoxy)-2-propanol. The reaction rate was 60 %, and the selectivity of hydrogenation was 90 %. Compound (2e) was isolated from the mixture by alumina column chromatography.

EXAMPLE 22

Synthesis of 2-Methyl-2-isobutyl-4-(1,3-dimethylbutoxy)-1,3-dioxolane (2e)

In a 0.5 ℓ autoclave were charged 50 g (0.287 mol) of compound (6e) obtained in Example 2-(1) and 1.0 g (1 %) of 5 % Pd/C (a dry preparation prepared by drying a 50 % preparation of a 5 % Pd-on-carbon powder, "E-type" (pH = 6.24) produced by N. E. Chemcat Corporation, in a vacuum pump at room temperature for 1 day). The mixture was heated with stirring under a hydrogen pressure of 10 kg/$cm^2$ and allowed to react at 190 °C for 6 hours under a hydrogen pressure of 20 kg/$cm^2$ to obtain a mixture composed of 2 % of compound (2e), 72 % of 3-(1,3-dimethylbutoxy)-1,2-propanediol, and 26 % of 1,3-bis-(1,3-dimethylbutoxy)-2-propanol. The reaction rate was 99 %, and the selectivity of hydrogenation was 90 %. Compound (2e) was isolated from the mixture by silica gel column chromatography.

EXAMPLE 23

Synthesis of 2,2-Diheptadecyl-4(1-heptadecyloctadecyloxymethyl)-1,3-dioxolane (2f)

(1) 2,2-Diheptadecyl-1,3-dioxolane-4-methanol (6f):

In a 500 mℓ reaction vessel equipped with a thermometer, a reflux condenser, a Dean-Stark trap, a calcium chloride cylinder, and a stirrer were charged 200 g (0.395 mol) of diheptadecyl ketone, 36.34 g (0.395 mol) of glycerin, 0.38 g (0.002 mol) of p-toluenesulfonic acid monohydrate, and 50 mℓ of hexane. The temperature was raised with stirring, and the mixture was allowed to react at 100 °C for 136 hours to remove a theoretical amount of water. Hexane was further removed by distillation to obtain 225.26 g of a reaction mixture containing compound (6f). The reaction rate was 85.4 % as measured by gas chromatography. A 124.31 g portion of the reaction mixture was dissolved in 600 mℓ of hexane, 1.2 g of sodium carbonate was added to the solution for neutralization, and the mixture was stirred 50 °C for 1 hour. Insoluble matters were removed by filtration at 50 °C, and the filtrate was cooled to room temperature. The thus precipitated unreacted diheptadecyl ketone (7.87 g) was recovered by filtration. The filtrate was cooled to -20 °C, and the precipitated crystals were collected by filtration and dried in a desiccator under reduced pressure to obtain 95.23 g (yield: 75.2 %) of compound (6f) (melting point: 37-39 °C).

(2) 2,2-Diheptadecyl-4-(1-heptadecyloctadecyloxymethyl)-1,3-dioxolane (2f):

In a 0.5 ℓ autoclave were added 50 g (0.086 mol) of compounds (6f) obtained in (1) above and 0.9 g (1.8 %) of 5 % Pd/C (a dry preparation prepared by drying a 50 % preparation of a 5 % Pd-on-carbon powder, "E-type" (pH = 6.24) produced by N. E. Chemcat Corporation, in a vacuum pump at room temperature for 1 day). The mixture was heated with stirring under a hydrogen pressure of 10 kg/$cm^2$ and allowed to react at 190 °C for 2 hours under a hydrogen pressure of 175 to 250 kg/$cm^2$. After completion of the reaction, the reaction mixture was filtered under reduced pressure by using Celite 545 as a filter aid to remove the catalyst. The filtrate was evaporated to obtain 43.33 g of a mixture containing compound (2f) (melting point: 36-38 °C). A 5 g portion of the mixture was dissolved in 20 mℓ of chloroform and purified by column chromatography on a column packed with 150 g of Wako Gel C-200. From the first fraction obtained with 1000 mℓ of chloroform was obtain 1.81 g (yield: 31.3 %) of compound (2f).

Melting Point: 55-56 °C

IR (neat, $cm^{-1}$): 2916, 2848 (C-H stretching), 1466, 1380 (C-H deformation), 1094 (C-O-C stretching)

EXAMPLE 24

Synthesis of 1,3-Bishexyloxy-2-propanol (3a)

(1) 2-Pentyl-1,3-dioxolane-4-methanol (6a) and 2-Pentyl-1,3-dioxan-5-ol (5a):

In a 3 ℓ reaction vessel equipped with a thermometer, a reflux condenser, a Dean-Stark trap, a calcium chloride cylinder, and a stirrer were charged 400 g (3.994 mol) of caproaldehyde, 404.5 g (4.392 mol) of glycerin, 3.80 g (0.02 mol) of p-toluenesulfonic acid monohydrate, and 400 mℓ of hexane. The temperature was raised with stirring, and the mixture was allowed to react at 69 to 77 °C for 4 hours to remove a theoretical amount of water. After completion of the reaction, the reaction mixture was cooled to 60 °C, and 4.24 g (0.04 mol) of sodium carbonate was added thereto for neutralization, followed by stirring at 60 °C for 30 minutes. The mixture was slowly heated up to 130 °C to recover 350 mℓ of hexane and to obtain 774.9 g of crude compounds (5a) and (6a).

The crude product was distilled under reduced pressure through a Vigreaux column (20 cm x 1.5 cm in diameter) to obtain 668.04 g (yield: 96.0 %) of compounds (5a) and (6a).

Boiling Point: 87-102 °C/0.3 mmHg

GC Purity: 94.1 %

Hydroxyl Value: 372.0 (theoretical value: 322.0)

(2) 1,3-Bishexyloxy-2-propanol (3a):

In a 0.5 ℓ autoclave were added 200 g (1.148 mol) of compounds (5a) and (6a) obtained in Example (1) above and 4 g (2 %) of 5 % Pd/C (a dry preparation prepared by drying a 50 % preparation of a 5 % Pd-on-carbon powder, "E-type" (pH = 6.9) produced by N. E. Chemcat Corporation, in a vacuum pump at room temperature for 1 day). The mixture was heated with stirring under a hydrogen pressure of 10 kg/cm$^2$ and allowed to react at 190 °C for 3 hours under a hydrogen pressure of 20 kg/cm$^2$ and then at the same temperature for 4 hours under a hydrogen pressure of 200 kg/cm$^2$. After completion of the reaction, the reaction mixture was filtered under pressure through a membrane filter (made of PTFE; pore size: 0.2 $\mu$m) to obtain 188.33 g (93.1 %) of a mixture composed of crude compound (3a) and 3-hexyloxy-1,2-propanediol (1a) (GC purity: 90 %). (3a)/(1a) = (3/7) (weight ratio).

To the mixture were added 200 mℓ of water, 200 mℓ of hexane, and 200 mℓ of methanol, followed by shaking. The lower layer was further extracted with 200 mℓ of hexane. The resulting hexane layer and the above-separated upper layer were combined and concentrated to obtain compound (3a).

EXAMPLE 25

Synthesis of 1,3-Bisheptyloxy-2-propanol (3b)

(1) 2-Heptyl-1,3-dioxolane-4-methanol (6b) and 2-heptyl-1,3-dioxan-5-ol (5b):

In a 3 ℓ reaction vessel equipped with a thermometer, a reflux condenser, a Dean-Stark trap, a calcium chloride cylinder, and a stirrer were charged 400 g (3.503 mol) of heptaldehyde, 354.84 g (3.853 mol) of glycerin, 3.33 g (0.0175 mol) of p-toluenesulfonic acid monohydrate, 200 mℓ of toluene, and 200 mℓ of hexane. The temperature was raised with stirring, and the mixture was allowed to react at 82 to 94 °C for 5 hours to remove a theoretical amount of water. After completion of the reaction, the reaction mixture was cooled to 60 °C, and 3.71 g (0.035 mol) of sodium carbonate was added thereto for neutralization, followed by stirring at 60 °C for 30 minutes. To the reaction mixture was added 100 mℓ of water, and the mixture stirred at 60 °C for 30 minutes, followed by allowing to stand for liquid-liquid separation. The upper layer was washed with two 100 mℓ portions of a saturated sodium chloride aqueous solution and evaporated to obtain 636.8 g of a mixture of crude compound (5b) and crude compound (6b). The mixture was distilled under reduced pressure through a Vigreaux column (20 cm x 1.5 cm in diameter) to obtain 591.21 g (89.6 %) of a mixture of compounds (5b) and (6b).

Boiling point: 87-102 °C/0.4 mmHg

GC Purity: 99.8 %

Hydroxyl value: 303.2 (theoretical value: 298.0)

(2) 1,3-Bisheptyloxy-2-propanol (3b):

In a 0.5 ℓ autoclave were charged 100 g (0.531 mol) of compounds (5b) and (6b) prepared in (1) above and 2 g (2 %) of 5 % Pd/C (a dry preparation prepared by drying a 50 % hydrous preparation of a 5 % Pd-on-carbon powder, "E-type" (pH = 6.9) produced by N. E. Chemcat Corporation, in a vacuum pump at room temperature for 1 day). The mixture was heated with stirring under a hydrogen pressure of 10 $kg/cm^2$, and allowed to react at 190 °C under a hydrogen pressure of 20 $kg/cm^2$ for 6 hours and then at the same temperature under a hydrogen pressure of 200 $kg/^2$ for 4 hours. After completion of the reaction, the reaction mixture was filtered through a membrane filter (made of PTFE; pore size: 0.2 μm) under pressure to obtain 88.91 g (yield: 88 %) of a mixture of crude compound (3b) and 3-heptyloxy-1,2-propanediol (1b) (GC purity: 90 %). (3b)/(1b) = 3/7 (weight ratio).

To the mixed product were added 100 mℓ of water, 100 mℓ of hexane, and 100 mℓ of methanol, and the mixture was shaken. The lower layer was removed, and the upper layer was further extracted 9 times with a mixture of 50 mℓ of water and 50 mℓ of methanol. The resulting upper layer contained concentrated compound (3b).

EXAMPLE 26

Synthesis of 1,3-Bisisopropoxy-2-propanol (3c)

In a 0.5 ℓ autoclave were added 136 g (1.029 mol) of solketal and 2.7 g (2 %) of 5 % Pd/C (a dry preparation prepared by drying a 50 % preparation of a 5 % Pd-on-carbon powder, "E-type" (pH = 6.24) produced by N. E. Chemcat Corporation, in a vacuum pump at room temperature for 1 day). The mixture was heated with stirring under a hydrogen pressure of 10 $kg/cm^2$, and allowed to react at 190 °C under a hydrogen pressure of 70 $kg/cm^2$ for 4 hours and then at the same temperature under a hydrogen pressure of 200 $kg/cm^2$ for 4 hours. After completion of the reaction, the reaction mixture was dissolved in isopropyl alcohol and filtered through 13.6 g of Kyowaad 600s under reduced pressure. The resulting isopropyl alcohol solution was evaporated to obtain 129.63 g (yield: 93.9 %) of a mixture of compound (3c) and 3-isopropoxy-1,2-propanediol (1c) (GC purity: 91.6 %). (3c)/(1c) = 37/63 (weight ratio).

A 129.32 g portion of the mixture was distilled under reduced pressure through a Vigreaux column (20 cm x 1.5 cm in diameter) to obtain 35.55 g of compound (3c) as a fraction at the boiling point of 52-53 °C/0.4 mmHg.

GC Purity: 99.5 %

Hydroxyl Value: 357.7 (theoretical value: 318.34)

EXAMPLE 27

Synthesis of 1,3-Bis(1-methylpropoxy)-2-propanol (3d)

(1) 2-Methyl-2-ethyl-1,3-dioxolane-4-methanol (6d):

In a 3 ℓ reaction vessel equipped with a thermometer, a reflux condenser, a Dean-Stark trap, a calcium chloride cylinder, and a stirrer were charged 400 g (5.547 mol) of methyl ethyl ketone, 561.91 g (6.102 mol) of glycerin, 21.10 g (0.1109 mol) of p-toluenesulfonic acid monohydrate, and 400 mℓ of hexane. The temperature was raised with stirring, and the mixture was allowed to react at 67 to 74 °C for 30 hours to remove a theoretical amount of water. After completion of the reaction, the reaction mixture was cooled to 60 °C, and 23.51 g (0.2218 mol) of sodium carbonate was added thereto for neutralization, followed by stirring at 60 °C for 30 minutes. The reaction mixture was gradually heated up to 120 °C to recover 400 mℓ of hexane. The reaction mixture was filtered to obtain 894.74 g of crude compound (6d), which was then distilled under reduced pressure through a Vigreaux column (20 cm x 1.5 cm in diameter) to obtain 725.14 g (yield: 89.4 %) of compound (6d).

Boiling point: 101-104 °C/17 mmHg

GC Purity: 99.1 %

Hydroxyl value: 381.8 (theoretical value: 383.8)

(2) 1,3-Bis(1-methylpropoxy)-2-propanol (3d):

In a 0.5 ℓ autoclave were charged 150 g (1.026 mol) of compound (6d) prepared in (1) above and 3 g (2 %) of 5 % Pd/C (a dry preparation prepared by drying a 50 % hydrous preparation of a 5 % Pd-on-carbon powder, "E-type" (pH = 6.9) produced by N. E. Chemcat Corporation, in a vacuum pump at room temperature for 1 day). The mixture was heated with stirring under a hydrogen pressure of 10 $kg/cm^2$, and allowed to react at 190 °C under a hydrogen pressure of 20 $kg/cm^2$ for 6 hours and then at the same temperature under a hydrogen pressure of 200 $kg/^2$ for 3 hours. After completion of the reaction, the reaction mixture was filtered through a membrane filter (made of PTFE; pore size: 0.2 µm) under pressure to obtain 141.76 g (yield: 93.2 %) of a mixture of crude compound (3d) and 3-(1-methylpropoxy)-1,2-propanediol (1d) (GC purity: 86.3 %). (3d)/(1d) = 3/7 (weight ratio).

The crude product was distilled under reduced pressure through a Vigreaux column (10 cm x 1.5 cm in diameter) to obtain 5.39 g of compound (3d) as a fraction at a boiling point of 63 °C/0.2 mmHg.

EXAMPLE 28

Synthesis of 1,3-Bis(1,3-dimethylbutoxy)-2-propanol (3e)

(1) 2-Methyl-2-isobutyl-1,3-dioxolane-4-methanol (6e):

In a 2 ℓ reaction vessel equipped with a thermometer, a reflux condenser, a Dean-Stark trap, a calcium chloride cylinder, and a stirrer were charged 790 g (7.887 mol) of methyl isobutyl ketone, 484.2 g (5.258 mol) of glycerin, 20.0 g (0.105 mol) of p-toluenesulfonic acid monohydrate, and 400 mℓ of hexane. The temperature was raised with stirring, and the mixture was allowed to react at 85 to 90 °C for 105 hours while removing a theoretical amount of water. After completion of the reaction, the reaction mixture was cooled to 40 °C, and 22.29 g (0.210 mol) of sodium carbonate was added thereto for neutralization, followed by stirring at 50 °C for 1 hour. To the mixture was added 300 mℓ of water, and the mixture was stirred at room temperature for 30 minutes and allowed to stand for liquid-liquid separation. The lower layer was removed, and the upper layer was washed twice with a saturated sodium chloride aqueous solution (250 mℓ/200 mℓ), dried over sodium sulfate, and evaporated to obtain 1050.1 g (crude yield: 93 %) of crude compound (6e) (purity: 81.1 %). The crude compound was distilled under reduced pressure to give 824.5 g (yield: 90.0 %) of compound (6e).

Boiling Point: 76 °C/1 mmHg
GC Purity: 97.3 %
Hydroxyl Value: 314.1 (theoretical value: 322.03)

(2) 1,3-Bis(1,3-dimethylbutoxy)-2-propanol (3e):

In a 0.5 ℓ autoclave were added 290 g (1.664 mol) of compounds (6e) obtained in (1) above and 2.9 g (1 %) of 5 % Pd/C (a dry preparation prepared by drying a 50 % preparation of a 5 % Pd-on-carbon powder, "E-type" (pH = 6.24) produced by N. E. Chemcat Corporation, in a vacuum pump at room temperature for 1 day). The mixture was heated with stirring under a hydrogen pressure of 10 $kg/cm^2$ and allowed to react at 190 °C under a hydrogen pressure of 70 $kg/cm^2$ for 11 hours and then at the same temperature under a hydrogen pressure of 200 $kg/cm^2$ for 10 hours. After completion of the reaction, the reaction mixture was filtered through 27 g of Kyowaad 600s to obtain 288.5 g (yield: 98.3 %) of a mixture composed of crude compound (3e) and 3-(1,3-dimethylbutoxy)-1,2-propanediol (1e) (GC purity: 90.6 %). (3e)/(1e) = 28/72 (weight ratio).

A 192.38 g portion of the mixture containing crude compound (3e) was purified by silica gel column chromatography (eluent: chloroform/methanol). The first fraction was distilled under reduced pressure through a Vigreaux column (12 cm x 1.5 cm in diameter) to yield 43.55 g of compound (3e).

Boiling Point: 94 °C/0.4 mmHg
GC Purity: 99.7 %
Hydroxyl Value: 213.1 (theoretical value: 215.44)

IR (neat, $cm^{-1}$): 3464 (O-H stretching), 2956, 2920, 2868 (C-H stretching), 1466, 1372 (C-H deformation), 1152, 1124 (C-O-C stretching), 1090 (C-O stretching)

NMR ($CDCℓ_3$, δ ppm): 0.86, 0.88, 0.89, 0.91 (12H, doublet, J = 4.2Hz, $-CH_2CH(CH_3)_2$), 1.11, 1.13 (6H, doublet, J = 5.8Hz, $-O-CH(CH_3)_2$), 1.10-1.25 (2H, doublet, $-CH_2CH(CH_3)_2$), 1.38-1.85 (4H, multiplet, $-CH_2CH(CH_3)_2$), 2.05-2.45 (1H, broad, -OH), 3.25-3.75

(6H, multiplet, >CHOC$H_2$C(OH)HC$H_2$OCH<), 3.77-3.93 (1H, multiplet, >CH-OH)

## EXAMPLE 29

### Synthesis of 1,3-Bis(1,3-dimethylbutoxy)-2-propanol (3e)

In a 0.5 ℓ autoclave were added 100 g (0.574 mol) of compound (6e) obtained in Example 28-(1) and 1.0 g (1 %) of 5 % Pd/C (a dry preparation prepared by drying a 50 % hydrous preparation of a 5 % Pd-on-carbon powder, "E-type" (pH = 6.24) produced by N. E. Chemcat Corporation, in a vacuum pump at room temperature for 1 day). The mixture was heated with stirring under a hydrogen pressure of 10 kg/cm$^2$ and allowed to react at 190°C for 4 hours under a hydrogen pressure of 70 kg/cm$^2$ to obtain a mixture composed of 7 % of compound (3e), 42 % of 3-(1,3-dimethylbutoxy)-1,2-propanediol, and 51 % of 2-methyl-2-isobutyl-4-(1,3-dimethlbutoxy)-1,3-dioxolane. The reaction rate was 60 %, and the selectivity of hydrogenation was 90 %. Compound (3e) was isolated from the mixture by silica gel column chromatography in the same manner as in Example 28.

## EXAMPLE 30

### Synthesis of 1,3-Bis(1,3-dimethylbutoxy)-2-propanol (3e)

In a 0.5 ℓ autoclave were added 50 g (0.287 mol) of compound (6e) obtained in Example 28-(1) and 1.0 g (1 %) of 5 % Pd/C (a dry preparation prepared by drying a 50 % preparation of a 5 % Pd-on-carbon powder, "E-type" (pH = 6.24) produced by N. E. Chemcat Corporation, in a vacuum pump at room temperature for 1 day). The mixture was heated with stirring under a hydrogen pressure of 10 kg/cm$^2$ and allowed to react at 190°C for 6 hours under a hydrogen pressure of 20 kg/cm$^2$ to obtain a mixture composed of 26 % of compound (3e), 72 % of 3-(1,3-dimethylbutoxy)-1,2-propanediol, and 2 % of 2-methyl-2-isobutyl-4-(1,3-dimethylbutoxy)-1,3-dioxolane. The reaction rate was 99 %, and the selectivity of hydrogenation was 90 %. Compound (3e) was isolated from the mixture by silica gel column chromatography in the same manner as in Example 28.

## EXAMPLE 31

### Synthesis of 1,3-Bis(1,3-dimethylbutoxy)-2-propanol (3e)

In a 0.5 ℓ autoclave were added 30 g (0.172 mol) of compound (6e) obtained in Example 28-(1) and 1.5 g (5 %) of 5 % Pd/C (a dry preparation prepared by drying a 50 % preparation of a 5 % Pd-on-carbon powder, "E-type" (pH = 6.24) produced by N. E. Chemcat Corporation, in a vacuum pump at room temperature for 1 day). The mixture was heated with stirring while introducing 5 ℓ/min of hydrogen at a hydrogen pressure of 20 kg/cm$^2$ and allowed to react at 190°C for 6 hours under a hydrogen pressure of 20 kg/cm$^2$ while introducing 5 ℓ/min of hydrogen to obtain a mixture composed of 26 % of compound (3e) and 74 % of 3-(1,3-dimethylbutoxy)-1,2-propanediol. The reaction rate was 100 %, and the selectivity of hydrogenation was 90 %. Compound (3e) was isolated from the mixture by silica gel column chromatography in the same manner as in Example 28.

## EXAMPLE 32

### Synthesis of 1,3-Bis(1,3-dimethylbutoxy)-2-propanol (3e)

In a 0.5 ℓ autoclave were added 30 g (0.172 mol) of compound (6e) obtained in Example 28-(1), 0.6 g (2 %) of 5 % Pd/C (a dry preparation prepared by drying a 50 % preparation of a 5 % Pd-on-carbon, "E-type" (pH = 6.24) produced by N. E. Chemcat Corporation, in a vacuum pump at room temperature for 1 day), and 6 g (0.0651 mol) of glycerin. The mixture was heated with stirring while introducing 2.5 ℓ/min of hydrogen at a hydrogen pressure of 20 kg/cm$^2$ and allowed to react at 190°C for 6 hours under a hydrogen pressure of 20 kg/cm$^2$ while introducing 2.5 ℓ/min of hydrogen to obtain a mixture composed of 17 % of compound (3e) and 83 % of 3-(1,3-dimethylbutoxy)-1,2-propanediol. The reaction rate was 100 %, and the selectivity of hydrogenation was 90 %. Compound (3e) was isolated from the mixture by silica gel column chromatography in the same manner as in Example 28.

EXAMPLE 33

Synthesis of 1,3-Bis(1,3-dimethylbutoxy)-2-propanol (3e)

(1) 2-Methyl-2-isobutyl-1,3-dioxolane-4-methanol (6e):

In a 2 ℓ reaction vessel equipped with a thermometer, a reflux condenser, a Dean-Stark trap, a calcium chloride cylinder, and a stirrer were charged 326.3 g (3.258 mol) of methyl isobutyl ketone, 200 g (2.172 mol) of glycerin, 8.3 g (0.0436 mol) of p-toluenesulfonic acid monohydrate, 200 mℓ of toluene, and 200 mℓ of hexane. The temperature was raised with stirring, and the mixture was allowed to react at 90 to 93 °C for 12 hours to remove a theoretical amount of water. After completion of the reaction, the reaction mixture was cooled to 60 °C, and 9.24 g (0.0872 mol) of sodium carbonate was added thereto for neutralization, followed by stirring at 60 °C for 30 minutes. The mixture was gradually heated up to 150 °C to recover hexane and toluene, followed by filtration. The filtrate was distilled under reduced pressure through a Vigreaux column (20 cm x 1.5 cm in diameter) to give 341 g (90.1 %) of compound (6e).

Boiling Point: 65-68 °C/0.5 mmHg
GC Purity: 98.9 %
Hydroxyl Value: 306.1 (theoretical value: 322.03)

(2) 1,3-Bis(1,3-dimethylbutoxy)-2-propanol (3e):

In a 0.5 ℓ autoclave were added 30 g (0.172 mol) of compounds (6e) obtained in (1) above, 1.5 g (5 %) of 5 % Pd/C (a dry preparation prepared by drying a 50 % preparation of a 5 % Pd-on-carbon powder, "K-type" (pH = 8.2) produced by N. E. Chemcat Corporation, in a vacuum pump at room temperature for 1 day), and 6.88 g (0.0747 mol) of glycerin. The mixture was heated with stirring under a hydrogen pressure of 10 kg/cm$^2$ while introducing 2.5 ℓ/min of hydrogen and allowed to react at 190 °C for 6 hours under a hydrogen pressure of 20 kg/cm$^2$ while introducing 2.5 ℓ/min of hydrogen to obtain a mixture composed of 14 % of crude compound (3e) and 86 % of 3-(1,3-dimethylbutoxy)-1,2-propanediol. The reaction rate was 100 %, and the hydrogenation selectivity was 90 %. Compound (3e) was isolated from the mixture by silica gel column chromatography in the same manner as in Example 28.

EXAMPLE 34

Synthesis of 1,3-Bis(1-heptadecyloctadecyloxy)-2-propanol (3f)

(1) 2,2-Diheptadecyl-1,3-dioxolane-4-methanol (6f):

In a 500 mℓ reaction vessel equipped with a thermometer, a reflux condenser, a Dean-Stark trap, a calcium chloride cylinder, and a stirrer were charged 200 g (0.395 mol) of diheptadecyl ketone, 36.34 g (0.395 mol) of glycerin, 0.38 g (0.002 mol) of p-toluenesulfonic acid monohydrate, and 50 mℓ of hexane. The temperature was raised with stirring, and the mixture was allowed to react at 100 °C for 136 hours to remove a theoretical amount of water. Hexane was further removed by distillation to give 225.26 g of a reaction mixture containing compound (6f). The reaction rate was 85.4 % as measured by gas chromatography. A 124.31 g portion of the reaction mixture was dissolved in 600 mℓ of hexane, 1.2 g of sodium carbonate was added to the solution for neutralization, and the mixture was stirred at 50 °C for 1 hour. The reaction mixture was filtered at 50 °C to remove any insoluble matter. The filtrate was cooled to room temperature, and the thus precipitated unreacted diheptadecyl ketone (7.87 g) was recovered. The filtrate was cooled to -20 °C, and the precipitated crystals were collected by filtration and dried in a desiccator under reduced pressure to obtain 95.23 g (yield: 75.2 %) of compound (6f) (melting point: 37-39 °C).

(2) 1,3-Bis(1-heptadecyloctadecyloxy)-2-propanol (3f):

In a 0.5 ℓ autoclave were added 50 g (0.086 mol) of compounds (6f) obtained in (1) above and 0.9 g (1.8 %) of 5 % Pd/C (a dry preparation prepared by drying a 50 % hydrous preparation of a 5 % Pd-on-carbon powder, "E-type" (pH = 6.24) produced by N. E. Chemcat Corporation, in a vacuum pump at room temperature for 1 day). The mixture was heated with stirring under a hydrogen pressure of 10 kg/cm$^2$ and allowed to react at 190 °C for 2 hours under a hydrogen pressure of 175 to 250 kg/cm$^2$. After completion of the reaction, the reaction mixture was filtered under reduced pressure using Celite 545 as a filter aid to

remove the catalyst. The filtrate was evaporated to obtain 43.33 g (yield: 86.4 %) of a mixture containing compound (3f) (m.p.: 36-38 °C). A 5 g portion of the resulting mixture was dissolved in 20 mℓ of chloroform, and the solution was passed through a column packed with 150 g of Wako Gel C-200 and eluted with chloroform. From the second fraction was obtained 0.74 g (yield: 12.8 %) of compound (1f).

Melting Point: 44-45 °C

IR (neat, $cm^{-1}$): 3496 (O-H stretching), 2920, 2852 (C-H stretching), 1466, 1378 (C-H deformation), 1118 (C-O-C stretching), 1094 (C-O stretching), 720 ($CH_2$, rocking)

EXAMPLE 35

Synthesis of 1,3-Bis(1-heptadecyloctadecyloxy)-2-propanol (3f)

(1) 2,2-Diheptadecyl-1,3-dioxolane-4-methanol (6f):

In a 1 ℓ reaction vessel equipped with a thermometer, a reflux condenser, a Dean-Stark trap, a calcium chloride cylinder, and a stirrer were charged 500 g (0.986 mol) of diheptadecyl ketone, 99.92 g (1.085 mol) of glycerin, 3.75 g (0.0197 mol) of p-toluenesulfonic acid monohydrate, and 160 mℓ of hexane. The temperature was raised with stirring, and the mixture was allowed to react at 100 °C for 190 hours to remove a theoretical amount of water, followed by cooling to 60 °C. To the reaction mixture was added 20.88 g (0.197 mol) of sodium carbonate, followed by stirring at 60 °C for 1 hour for neutralization. The reaction mixture was filtered to remove 35.29 g of the insoluble matter. The filtrate was cooled to room temperature, and the thus precipitated unreacted diheptadecyl ketone (36.32 g) was recovered by filtration. The filtrate was cooled to -20 °C, and the precipitated crystals were collected by filtration and dried in reduced pressure to obtain 487.28 g (yield: 85.1 %) of compound (6f) (melting point: 37-39 °C).

(2) 1,3-Bis(1-heptadecyloctadecyloxy)-2-propanol (3f):

In a 1 ℓ autoclave were charged 200 g (0.344 mol) of compounds (6f) obtained in (1) above and 3.8 g (1.9 %) of 5 % Pd/C (a dry preparation prepared by drying a 50 % hydrous preparation of a 5 % Pd-on-carbon powder, "E-type" (pH = 6.24) produced by N. E. Chemcat Corporation, in a vacuum pump at room temperature for 1 day). The mixture was heated with stirring under a hydrogen pressure of 10 $kg/cm^2$ and allowed to react at 190 °C for 15 hours under a hydrogen pressure of 200 $kg/cm^2$. After completion of the reaction, the reaction mixture was filtered under reduced pressure using Kyowaad 600s as a filter medium to separate 36.17 g of solids (the catalyst and diheptadecyl ketone), and the filtrate was evaporated to recover 151.11 g of a solid. A 150 g portion of the solid was dissolved in 500 mℓ of ethanol, and the solution was charged in a 1 ℓ four-necked flask equipped with a thermometer, a reflux condenser, and a stirring rod together with 15 g of 36 % HCℓ and 135 g of water. The mixture was heat-refluxed at 80 °C for 24 hours while stirring. After cooling, the formed precipitate (135.18 g) was collected by filtration and crystallized from hexane to recover 77.72 g of diheptadecyl ketone by filtration. The crystallization mother liquor was concentrated to obtain 57.08 g of a mixture (A) of compound (3f) and 3-(1-heptadecyloctadecyloxy)-1,2-propanediol.

The mother liquor of the reaction mixture (after removal of the insoluble matter) was evaporated to remove ethanol, and the residue was re-dissolved in 200 mℓ of hexane, washed successively with water and a saturated sodium hydrogencarbonate aqueous solution, dried over sodium sulfate, and evaporated to obtain 5.85 g of a mixture (B) of compound (3f) and 3-(1-heptadecyloctadecyloxy)-1,2-propanediol.

Mixtures (A) and (B) were combined (amounting to 62.93 g) and dissolved in 100 mℓ of chloroform, and the solution was passed through a column packed with 400 g of Wako Gel C-200 and eluted with chloroform to recover 15.81 g of compound (3f).

The glycerin derivatives according to the present invention have hydrophilic properties and hygroscopic properties on account of their polar group, such as a hydroxyl group. Therefore, they exhibit satisfactory adsorbability to the skin or satisfactory compatibility to polar group-containing oils. Unlike the known glyceryl ethers, such as comparative compounds (a) and (b) shown in Table 3, which provide W/O emulsions, the glycerin derivatives of the present invention, while having approximately the same number of carbon atoms as the known glyceryl ethers, provide O/W emulsions because of the branching at the α-position. That is, the glyceryl derivatives of the present invention can be said to be oil having very high polarity. Further, they hardly undergo association of liquid crystals and are hardly dissolved in water to apparently assume the character of oil. Accordingly, the compounds of the present invention are useful as highly durable emollient or moisturizing agent which, when applied to the skin, exhibit high resistance

against washing and remain on the skin long. Further, they are applicable as lubricants, taking advantage of their compatibility with other polar oils.

While the invention has been described in detail and with reference to specific examples thereof, it will be apparent to one skilled in the art that various changes and modifications can be made therein without departing from the spirit and scope thereof.

## Claims

1. A glycerin derivative represented by formula (1A):

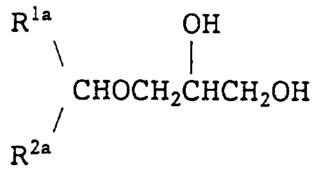

(1A)

wherein $R^{1a}$ and $R^{2a}$, which may be the same or different, each represent an alkyl group having from 2 to 21 carbon atoms.

2. A process for producing a glycerine derivative represented by formula (1):

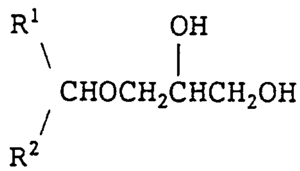

(1)

wherein $R^1$ represents a hydrogen atom or an alkyl group having from 1 to 21 carbon atoms; $R^2$ represents an alkyl group having from 1 to 21 carbon atoms; or $R^1$ and $R^2$ are taken together to form an alkylene group having from 2 to 21 carbon atoms; provided that $R^1$ and $R^2$ do not simultaneously represent an alkyl group having no hydrogen atom at the $\alpha$-position thereof,
comprising reacting a carbonyl compound represented by formula (4):

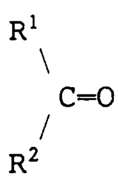

(4)

wherein $R^1$ and $R^2$ are as defined above,
with glycerin in the presence of an acid catalyst and hydrogenating the resulting compound.

3. The process of claim 2, wherein said hydrogenation is carried out in the presence of a palladium catalyst.

4. The process of claim 3, wherein said catalyst is supported on carbon.

5. The process of claim 4, wherein said catalyst has a pH of from 5 to 8.

6. A glycerin derivative represented by formula (2A):

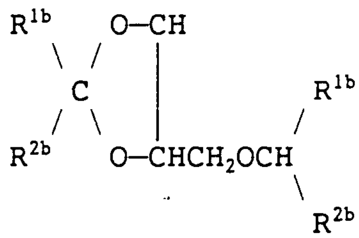

(2A)

wherein $R^{1b}$ and $R^{2b}$, which may be the same or different, each represent an alkyl group having from 3 to 21 carbon atoms.

**7.** A process for producing a glycerin derivative represented by formula (2):

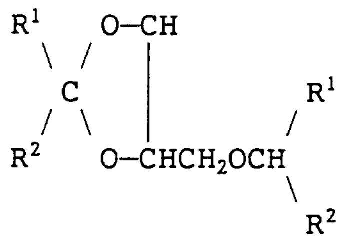

(2)

wherein $R^1$ represents a hydrogen atom or an alkyl group having from 1 to 21 carbon atoms; $R^2$ represents an alkyl group having from 1 to 21 carbon atoms; or $R^1$ and $R^2$ are taken together to form an alkylene group having from 2 to 21 carbon atoms; provided that $R^1$ and $R^2$ do not simultaneously represent an alkyl group having no hydrogen atom at the $\alpha$-position thereof,
comprising reacting a carbonyl compound represented by formula (4):

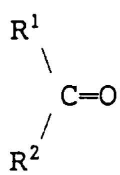

(4)

wherein $R^1$ and $R^2$ are as defined above,
with glycerin in the presence of an acid catalyst and hydrogenating the resulting compound.

**8.** The process of claim 7, wherein said hydrogenation is carried out in the presence of a palladium catalyst.

**9.** The process of claim 8, wherein said catalyst is supported on carbon.

**10.** The process of claim 9, wherein said catalyst havs a pH of from 5 to 8.

**11.** A glycerin derivative represented by formula (3A):

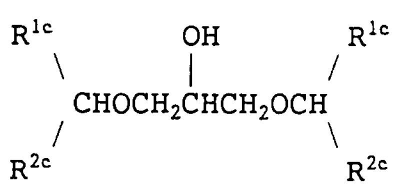

(3A)

wherein $R^{1c}$ represents an alkyl group having from 1 to 21 carbon atoms; and $R^{2c}$ represents an alkyl group having from 3 to 21 carbon atoms.

**12.** A process for producing a glycerin derivative represented by formula (3):

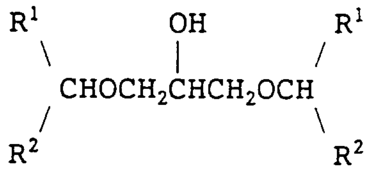

(3)

wherein $R^1$ represents a hydrogen atom or an alkyl group having from 1 to 21 carbon atoms; $R^2$ represents an alkyl group having from 1 to 21 carbon atoms; or $R^1$ and $R^2$ are taken together to form an alkylene group having from 2 to 21 carbon atoms; provided that $R^1$ and $R^2$ do not simultaneously represent an alkyl group having no hydrogen atom at the α-position thereof,
comprising reacting a carbonyl compound represented by formula (4):

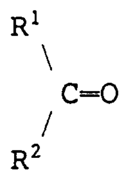

(4)

wherein $R^1$ and $R^2$ are as defined above,
with glycerin in the presence of an acid catalyst and hydrogenating the resulting compound.

**13.** The process of claim 12, wherein said hydrogenation is carried out in the presence of a palladium catalyst.

**14.** The process of claim 13, wherein said catalyst is supported on carbon.

**15.** The process of claim 14, wherein said catalyst havs a pH of from 5 to 8.

European Patent Office

# EUROPEAN SEARCH REPORT

Application Number
EP 94 10 7283

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | US-A-2 156 724 (T EVANS ET AL)<br>* the whole document * | 1,11 | C07C41/01<br>C07C41/28<br>C07C43/13<br>C07D317/22 |
| Y | WO-A-93 02033 (HENKEL)<br>* page 3, paragraph 3; claims; examples * | 2,12 | |
| Y | DE-A-32 20 035 (KAO SOAP)<br>* page 12, paragraph 1 * | 2,12 | |
| A | DE-A-29 45 918 (BASF)<br>* example * | 2,7,12 | |
| A | EP-A-0 133 881 (CHEMISCHE WERKE HÜLS)<br>* the whole document * | 2,7,12 | |
| A | US-A-4 088 700 (TEXACO DEVELOPMENT)<br>* the whole document * | 2,7,12 | |

TECHNICAL FIELDS SEARCHED (Int.Cl.5)

C07C
C07D

The present search report has been drawn up for all claims

| Place of search | Date of completion of the search | Examiner |
|---|---|---|
| THE HAGUE | 7 July 1994 | Heywood, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)